(19)

(11) **EP 3 576 866 B1**

(12) # EUROPÄISCHE PATENTSCHRIFT

(45) Veröffentlichungstag und Bekanntmachung des Hinweises auf die Patenterteilung:
**01.05.2024 Patentblatt 2024/18**

(21) Anmeldenummer: **18705839.1**

(22) Anmeldetag: **31.01.2018**

(51) Internationale Patentklassifikation (IPC):
***B01D 67/00*** *(2006.01)* ***B01D 69/08*** *(2006.01)*
***B01D 69/14*** *(2006.01)* ***B01D 71/68*** *(2006.01)*

(52) Gemeinsame Patentklassifikation (CPC):
**B01D 69/08; B01D 67/0011; B01D 67/0016; B01D 69/144; B01D 71/68;** B01D 2323/2187

(86) Internationale Anmeldenummer:
**PCT/EP2018/052378**

(87) Internationale Veröffentlichungsnummer:
**WO 2018/141780 (09.08.2018 Gazette 2018/32)**

(54) **HOHLFASERMEMBRAN MIT VERBESSERTER BIOKOMPATIBILITÄT UND VERRINGERTER ELUTION HYDROPHILER POLYMERE**

HOLLOW-FIBRE MEMBRANE WITH IMPROVED BIOCOMPATIBILITY AND REDUCED ELUTION OF HYDROPHILIC POLYMERS

MEMBRANE À FIBRES CREUSES OFFRANT UNE BIOCOMPATIBILITÉ AMÉLIORÉE ET ÉLUTION RÉDUITE DE POLYMÈRES HYDROPHILES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.02.2017 DE 102017201630**

(43) Veröffentlichungstag der Anmeldung:
**11.12.2019 Patentblatt 2019/50**

(60) Teilanmeldung:
**24166993.6**

(73) Patentinhaber: **Fresenius Medical Care Deutschland GmbH**
**61352 Bad Homburg (DE)**

(72) Erfinder:
- **PAUL, Michael**
  **Lebach 66822 (DE)**
- **FISLAGE, Rainer**
  **St. Wendel 66606 (DE)**
- **HANSEL, Dietmar**
  **Ottweiler 66564 (DE)**

(74) Vertreter: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

(56) Entgegenhaltungen:
**EP-A1- 0 749 775** **EP-A1- 2 719 408**
**EP-A1- 2 737 916** **EP-A1- 2 987 514**
**WO-A2-2012/106583**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

### Thema der Erfindung

Die Erfindung betrifft ein Verfahren zur Herstellung einer Hohlfasermembran sowie damit hergestellte Holfasermembranen, die ein Membranmaterial aufweisen, das ein hydrophobes und ein hydrophiles Polymer umfasst und verbesserte Biokompatibilitätseigenschaften, insbesondere verbesserte Eigenschaften bezüglich der C5a Aktivierung und des "Platelet Loss", aufweist.

### Hintergrund der Erfindung

Hohlfasermembranen finden in der Aufreinigung von Flüssigkeiten weiträumig Verwendung. Insbesondere werden Hohlfasermembranen in der Medizintechnik für die Blutreinigung in der Dialyse von nierenkranken Patienten eingesetzt. Hohlfasermembranen werden in Form von Hohlfasermembranbündeln in Filtermodulen verbaut, die in der extrakorporalen Blutbehandlung eingesetzt werden. Die Herstellung solcher Filtermodule zur Blutreinigung, sogenannter Dialysatoren, erfolgt im Massenproduktionsmaßstab.

Die für die Blutreinigung verwendeten Hohlfasermembranen bestehen häufig aus einem hydrophoben und einem hydrophilen Polymer, insbesondere aus Polysulfon und Polyvinylpyrrolidon, da sich diese Materialien als besonders hämokompatibel herausgestellt haben und daher in der Blutbehandlung, insbesondere in der Hämodialyse aus medizinischer Sicht zu bevorzugen sind. Unter "*Polysulfon*" wird im Sinne der vorliegenden Anmeldung ein Polymer verstanden, dass eine Sulfongruppe in der Haupt- oder Nebenkette des Polymers aufweist. Typische Vertreter von Polysulfonen sind Polysulfon auf der Basis Bisphenol-A (PSU), Polyethersulfon (PES), Polyphenylsulfon und Copolymere enthaltend Sulfongruppen. Weitere Vertreter von Polysulfonpolymeren sind im Stand der Technik bekannt und im Sinne der vorliegenden Anmeldung für die Herstellung von Blutbehandlungsmembranen geeignet. Unter "*Polyvinylpyrrolidon*" wird ein Polymer verstanden, das unter Verwendung des Monomeren Vinylpyrrolidon oder Derivaten davon hergestellt wird. Weitere geeignete hydrophobe Polymere sind Polyamid, Polyacrylnitril und regenerierte Cellulose und Derivate der Cellulose. Ein weiteres geeignetes hydrophiles Polymer ist Polyethylenglycol.

Die grundsätzlichen Prinzipien der Herstellung von Hohlfasermembranen und deren Herstellung sind im Stand der Technik beschrieben:

- Marcel Mulder; Principles of Membrane Technology; Kluwer Academic Publisher 1996; Kapitel III, Preparation of synthetic membranes
- EP 0 168 783

Nach den im Stand der Technik beschriebenen Herstellmethoden von Hohlfasermembranen wird eine Spinnlösung bereitgestellt, die ein hydrophobes Polymer auf Basis von Polysulfon, und ein hydrophiles Polymer auf Basis von Vinylpyrrolidon, insbesondere Polyvinylpyrrolidon, und ein oder mehrere Lösungsmittel und gegebenenfalls Additive umfasst. Als Lösungsmittel werden gängigerweise polare aprotische Lösungsmittel verwendet, insbesondere Dimethylacetamid (DMAc), N-Methylpyrrolidon (NMP), Dimethylformamid (DMF) oder Dimethylsulfoxid (DMSO). Die Bezeichnung Lösungsmittel bezieht sich dabei auf die Lösungseigenschaften des Lösungsmittels gegenüber den eingesetzten Polymeren, insbesondere auf Polysulfon und Polyvinylpyrrolidon. Zusätze von Additiven, z.B. polaren protischen Flüssigkeiten, wie z.B. Wasser, können ebenfalls in geringen Anteilen Bestandteile der Spinnlösung sein. Auch Mischungen von Lösungsmitteln sind dem Stand der Technik nach bekannt.

Die Spinnmasse wird über einen kreisförmigen konzentrischen Ringspalt einer Düse gesponnen. Die Spinndüse weist weiterhin eine zentrale Bohrung auf, durch die ein Koagulationsmittel geleitet wird. Das Koagulationsmittel besteht üblicherweise aus einer Mischung eines aprotischen polaren Lösemittels, wie z.B. DMAc und einer protischen Flüssigkeit, wie z.B. Wasser. Spinnmasse und Koagulationsmittel werden durch den Ringspalt und die zentrale Bohrung der Spinndüse zu einem Spinnfaden verarbeitet, in dessen Lumen sich das Koagulationsmittel befindet. Der Spinnfaden wird danach in der Regel durch eine Luftstrecke geführt, in der die Spinnmasse des Spinnfadens zu koagulieren beginnt und ein zweiphasiges System einer Gel- und einer Solphase ausbildet. Anschließend wird der Spinnfaden in ein Fällbad eingeleitet, in dem sich ein Fällmittel befindet. Durch Einleiten in das Fällbad bildet sich aus dem Spinnfaden die Hohlfasermembranstruktur aus. Als Fällmittel dienen in der Regel Wasser oder Gemische aus protischen und aprotischen Lösungsmitteln, insbesondere Wasser und Dimethylacetamid, N-Metyhlpyrrolidon, Dimethylformamid oder Dimethylsulfoxid. Die erhaltene Hohlfasermembran wird anschließend durch Spülbäder geführt und getrocknet und auf eine Haspel aufgenommen. Von der Haspel können die Hohlfasermembranen in Form von Hohlfaserbündeln entnommen werden. Für den Bau von Hohlfasermembranfiltern werden solche Hohlfasermembranbündel in ein Gehäuse, vorzugsweise ein zylindrisches Gehäuse, eingebracht. Die Enden des Hohlfasermembranbündels werden in eine Vergussmasse eingebettet und die offenen Enden der Hohlfasern freigelegt. Die Vergussmasse bildet einen abdichtenden Bereich zwischen dem Inneren der Hohlfasermembranen, dem Gehäuse und den die Hohlfasermembranen umgebenden Bereich. Es wird dadurch im fertigen Hohlfasermembranfilter ein erster Raum gebildet, der An- und Abströmbereiche der Enden des Hohlfasermembranbündels und auch das Innere der Hohlfasermembranen umfasst. Ein zweiter Raum bildet sich demgemäß durch den Bereich im Zwischenraum zwischen den Hohlfasermembranen und zwischen Gehäusewand und den Hohlfasermembranen. Fluidzugänge am Gehäuse des Hohlfasermembranfilters ermöglichen das Zuführen und Abführen von Flüssigkeiten und Fluiden zu dem ersten und/ oder dem zweiten Raum des Hohlfasermembranfilters.

Die einzelnen Herstellschritte sind entscheidend, um Hohlfasermembranen mit vorbestimmten Leistungseigenschaften und Abtrennverhalten herstellen zu können. Im Wesentlichen sind die Abtrennleistung und die Trennschärfe der Hohlfasermembranen in einer Filtrationsanwendung einer extrakorporalen Blutbehandlung entscheidend für die jeweilige therapeutische Anwendung. Es ist daher in einem Herstellverfahren wichtig, die Herstellschritte so anzupassen, dass die angestrebten Leistungseigenschaften der Hohlfasermembranen, die für die jeweilige therapeutische Behandlung notwendig sind, erreicht werden. Daneben nimmt das Herstellverfahren aber auch entscheidenden Einfluss auf die Biokompatibilität der Hohlfasermembranen. Unter Biokompatibilität wird hierbei die physiologische Verträglichkeit eines Dialysators in einer extrakorporalen Blutbehandlung eines Patienten verstanden. Insbesondere wird eine Hohlfasermembran oder ein entsprechender Hohlfasermembranfilter (im Weiteren auch als Dialysator bezeichnet) als biokompatibel verstanden, wenn er im Blutkontakt der extrakorporalen Blutbehandlung keine oder nur geringe beeinträchtigende Reaktionen hervorruft. Solche Reaktionen können durch Wechselwirkung der Hohlfasermembranoberfläche mit in Kontakt stehenden Blutbestandteilen hervorgerufen werden. Dies sind insbesondere Wechselwirkungen mit Blut auf zellulärer Basis, aber auch Wechselwirkungen mit im Blutplasma vorhandenen Proteinen.

Zur Beurteilung der Biokompatibilität von handelsüblichen Dialysatoren wird nach einer Methode Vienken et al. (A. Erlenkötter, P. Endres, B. Nederlof, C. Hornig, J. Vienken; Artificial Organs; 32 (12), 962, (2008)) vorgeschlagen, Testblut durch einen Dialysator für einen vorbestimmten Zeitraum rezirkulieren zu lassen und die auftretenden Nebenreaktionen anhand einer Auswahl von sogenannten Hämokompatibilitätsmarkern zu erfassen. Als Hämokompatibilitätsmarker wurden nach dieser Methode der Komplementfaktor 5a (C5a), der Thrombin-Antithrombin III-Komplex (TAT), die Thrombozytenzahl ("platelet count"= PLT), der Plättchenfaktor 4 ("platelet factor 4"= PF4) und die Freisetzung von Elastase aus polymorphkernigen Granulozyten (PMN-Elastase) herangezogen.

Zur Beurteilung der Biokompatibilität eines Dialysators wird nach dieser Methode vorgeschlagen, die einzelnen Hämokompatibilitätsmarker nach einem "Scoring"-System zu bewerten. Es wird weiterhin vorgeschlagen, einen Gesamt-Hämokompatibilitätswert ("Total Hemocompatibility Score"= THS) für einen Dialysator aus dem Scoring der einzelnen Hämokompatibilitätsmarker zu errechnen und so unterschiedliche Dialysatoren hinsichtlich ihrer Biokompatibilität vergleichbar zu machen.

Im Ergebnis dieser Methode wurde deutlich, dass sowohl das Membranmaterial, wie auch die unterschiedlichen Sterilisationsmethoden, mit denen handelsübliche Dialysatoren sterilisiert werden, offensichtlich einen Einfluss auf die Biokompatibilität der Hohlfasermembranen haben können. Insbesondere wurde ein Unterschied der Biokompatibilität der untersuchten Dialysatoren aufgrund der unterschiedlichen Membranmaterialien Polysulfon, Polyethersulfon/Polyarylate, regenerierte Cellulose, veresterte Cellulose und der unterschiedlichen Sterilisationsmethoden, wie Dampfsterilisation, Strahlensterilisation ($\gamma$-Strahlen oder Elektronenstrahlen) und Vakuum-Dampf Sterilisation, festgestellt.

Hohlfasermembranen und Dialysatoren werden als medizinische Einwegartikel in der therapeutischen Blutbehandlung verwendet und entsprechend als Massenartikel für die Versorgung von Patienten im Handel bereitgestellt. Die Herstellmethode der Hohlfasermembranen und der Dialysatoren erfolgt daher oftmals nach wirtschaftlichen Interessen und Aspekten der Produktivität. D.h. dass die Herstellung von Hohlfasermembranen und Dialysatoren zwar so ausgerichtet ist, dass die erforderlichen Leistungseigenschaften erreicht werden, daneben die Herstellung aber nach möglichst kostengünstigen Maßstäben ausgerichtet ist. Die Untersuchungen von Vienken et al. machen dabei deutlich, dass die im Stand der Technik etablierten Herstellverfahren von Hohlfasermembranen und Dialysatoren hinsichtlich einer vorteilhaften anzustrebenden Biokompatibilität häufig nicht optimal verläuft.

Im Stand der Technik sind Herstellmethoden von Hohlfasermembranen mit dem Ziel beschrieben, Hohlfasermembranen bereitzustellen, die bei vorgegebener Trennleistung eine hohe Biokompatibilität bei gleichzeitiger kostengünstiger Herstellung ermöglichen. Insbesondere sind diesbezüglich Herstellverfahren von Hohlfasermembranen beschrieben, bei denen die Hohlfasermembranen mit einem fettlöslichen Vitamin, z.B. einem Vitamin E, modifiziert werden. Es wird beschrieben, dass derartige Modifikationen z.B. durch Zugabe von Vitamin E in das im Herstellverfahren verwendete Koagulationsmittel erfolgen können. Auf diese Weise soll eine Beschichtung der inneren Oberfläche der hergestellten Hohlfasermembranen mit Vitamin E und somit eine verbesserte Biokompatibilität erreicht werden. Es wird dabei vermutet, dass die mit Vitamin E modifizierten Hohlfasermembranen, wenn sie in Kontakt mit Behandlungsblut kommen, eine antioxidative Wirkung auf Blutzellen haben bzw. die Wirkung des immunologisch relevanten "chemical burst" vermindern und allgemein den pro-oxidativen Blutstatus von Patienten mit chronischem Nierenversagen korrigieren.

EP 0 850 678 B1 beschreibt in diesem Zusammenhang ein Herstellverfahren von Hohlfasermembranen auf Polysulfon- und Polyvinylpyrrolidon-Basis, wobei dem Koagulationsmittel ein Tensid und ein Vitamin E zugesetzt wird.

Das Verfahren zielt darauf ab, Vitamin E auf der inneren Oberfläche in der Herstellung einer Hohlfasermembran abzuscheiden. Die Blutkompatibilität der inneren Oberfläche der Hohlfasermembran soll durch die hydrophobe Wirkung des Vitamin E werden und so eine antioxidative Wirkung gegenüber Blutzellen erreicht werden.

Auf der anderen Seite wird die Hydrophilisierung der inneren Oberfläche von Polysulfon-Hohlfasermembranen im Zusammenhang mit einer besseren Blutbenetzbarkeit und einer besseren Biokompatibilität diskutiert. In diesem Zusammenhang beschreibt die EP 0 568 045 eine Herstellung einer Hohlfasermembran auf Basis von Polysulfon. Die Herstellung der Hohlfasermembran erfolgt mit Hilfe eines Koagulationsmittels, das 0,5 bis 4 % eines Polyvinylpyrrolidons enthält. Dabei bewirkt der Zusatz von Polyvinylpyrrolidon in dem Koagulationsmittel eine Erhöhung des Anteils von Polyvinylpyrrolidon auf der inneren Oberfläche der hergestellten Hohlfasermembran.

Generell nachteilig ist, dass Polyvinylpyrrolidon mit hohem Molekulargewicht vom menschlichen Organismus schlecht oder gar nicht metabolisiert werden kann und vom menschlichen Körper nur bedingt über die Nieren ausgeschieden werden kann. Infolgedessen wird eine Kumulation von Poylvinylpyrrolidon mit hohem Molekulargewicht im Körper von chronischen Dialysepatienten beobachtet. EP2987514A1, EP2737916A1, EP0749775A1 und EP2719408A1 offenbaren Hohlfasermembranen welche Vitamin E oder $\alpha$-Tocopherol beinhalten.

**Aufgabe der Erfindung**

Hinsichtlich der im Stand der Technik vorherrschenden Probleme hat sich gezeigt, dass das Bedürfnis nach einer Bereitstellung von Hohlfasermembranen mit einer verbesserten Biokompatibilität besteht. Insbesondere besteht weiterhin das Bedürfnis, Herstellmethoden zu finden, die eine hervorragende Biokompatibilität von sterilen Hohlfasermembranen oder Dialysatoren gewährleistet. Insbesondere soll die Herstellmethode dabei aber kostengünstig und unter Einsparung von Material- und Anlagenaufwand erreicht werden.

In einem **ersten Aspekt** der Erfindung bestand daher die Aufgabe, ein Verfahren zur Herstellung von Hohlfasermembranen bereitzustellen, das die Herstellung von Hohlfasermembranen mit einer verbesserten Biokompatibilität gegenüber Behandlungsblut ermöglicht und das insbesondere die Herstellung von Hohlfasermembranen mit einer geringeren Komplementaktivierung und einem geringeren Thrombozytenverlust gegenüber Behandlungsblut möglich macht. Weiterhin bestand im ersten Aspekt der Erfindung die Aufgabe, ein derartiges Herstellverfahren für Hohlfasermembranen mit verbesserter Biokompatibilität bereitzustellen, das kostengünstig ist und durch geringen Anlagen- und Materialaufwand bereitgestellt werden kann.

In **weiteren Aspekten** der Erfindung stellte sich die Aufgabe, eine Hohlfasermembran mit hoher Biokompatibilität für die Blutbehandlung bereitzustellen. Insbesondere stellt sich nach dem zweiten Aspekt der Erfindung die Aufgabe, eine Hohlfasermembran bereitzustellen, die durch eine niedrige Komplementaktivierung und eine geringe Neigung zum Thrombozytenverlust, dem sogenannten "Platelet Loss", gegenüber Behandlungsblut charakterisiert ist. Weiterhin bestand die Aufgabe, derartige Hohlfasermembranen kostengünstig und mit geringem Herstellaufwand bereitstellen zu können.

**Zusammenfassung der Erfindung**

Die zugrundeliegende Aufgabe wird gelöst durch das erfindungsgemäße Verfahren zu Herstellung einer Hohlfasermembran, wobei ein nicht wasserlösliches Antioxidans, insbesondere ein fettlösliches Vitamin, weiter insbesondere $\alpha$-Tocopherol oder ein Tocotrienol in der Spinnmasse vorgelegt wird und im Koagulationsmittel ein hydrophiles Polymer vorgelegt wird. Das erfindungsgemäße Verfahren wird in Anspruch 1 als ersten Aspekt definiert.

In einer weiteren Ausführungsform des ersten Aspekts ist das Verfahren dadurch gekennzeichnet, dass die Spinnmasse 2 bis 7 Gew.%, insbesondere 3 bis 5 Gew.% Polyvinylpyrrolidon bezogen auf die Gesamtmasse der Spinnmasse enthält.

In einer weiteren Ausführungsform des ersten Aspekts ist das Verfahren dadurch gekennzeichnet, dass das Koagulationsmittel 25 % bis 60 Gew.% eines polar aprotischen Lösungsmittels, insbesondere DMAc, und 40 bis 75 Gew.% eines polar protischen Nicht-Lösemittels, insbesondere Wasser, enthält.

In einer weiteren Ausführungsform des ersten Aspekts ist das Verfahren dadurch gekennzeichnet, dass das im Koagulationsmittel enthaltene hydrophile Polymer, insbesondere das Poylvinylpyrrolidon, eine Molekulargewichtsverteilung im Bereich von 200.000 g/mol bis 2.000.000 g/mol, insbesondere ein gewichtsmittleres Molekulargewicht von 900.000 g/mol aufweist.

In einer weiteren Ausführungsform des ersten Aspekts ist das Verfahren dadurch gekennzeichnet, dass die Ringspaltdüse auf eine Temperatur von 30°C bis 85°C, insbesondere von 65°C bis 85°C temperiert ist.

In einer weiteren Ausführungsform des ersten Aspekts ist das Verfahren dadurch gekennzeichnet, dass das Fällbad auf eine Temperatur von 50°C bis 85°C temperiert ist.

In einer weiteren Ausführungsform des ersten Aspekts ist das Verfahren dadurch gekennzeichnet, dass die Abzugsgeschwindigkeit des Spinnfadens 100 mm/s bis 1500 mm/s beträgt.

In einer weiteren Ausführungsform des ersten Aspekts ist das Verfahren dadurch gekennzeichnet, dass der Spinnfaden nach der Spinndüse einen Fällspalt von 50 mm bis 1500 mm durchläuft, bevor er in das Fällbad eingeleitet wird.

In einer weiteren Ausführungsform des ersten Aspektes ist das Verfahren dadurch gekennzeichnet, dass das hydrophile Polymer der Spinnmasse Polyvinylpyrrolidon (PVP) umfasst und dass das hydrophile Polymer im Koagulationsmittel Polyvinylpyrrolidon (PVP) umfasst, wobei das gewichtsmittlere Molekulargewicht (Mw) des PVP im Koagulationsmittel höher ist als das des PVP in der Spinnmasse.

In einer weiteren Ausführungsform des ersten Aspektes ist das Verfahren dadurch gekennzeichnet, dass das gewichtsmittlere Molekulargewicht (Mw) des PVP in der Spinnmasse unter 1.000.000 g/mol beträgt und das gewichtsmittlere Molekolargewicht (Mw) des PVP in dem Koagulationsmittel über 1.000.000 g/mol beträgt.

Das im Rahmen der vorliegenden Anmeldung mehrfach angegebene gewichtsmittlere Molekulargewicht für PVP wird auf übliche Weise mittels Lichtstreuung (typischerweise im Rahmen einer GPC-LS-Messung) bestimmt. Hierzu wird beispielsweise auf die Seite 37 der Broschüre "Volker Bühler - Kollidon® Polyvinylpyrrolidone excipients for the pharmaceutical industry, 9. Auflage (March 2008)" der BASF AG und die dort angegebene Literatur verwiesen.

Ein zweiter Aspekt der Anmeldung betrifft die Bereitstellung einer Hohlfasermembran. Die erfindungsgemäße Membran wird in Anspruch 6 definiert.

In einer weiteren Ausführungsform gemäß dem zweiten Aspekt ist die Hohlfasermembran dadurch gekennzeichnet, dass die Elution des hydrophilen Polymers, insbesondere des Polyvinylpyrrolidon, nach einem Lagerzeitraum von 30 Tagen bei 80°C und < 5 % relativer Luftfeuchte in einem Elutionstest weniger als $4000*10^{-7}$ mg pro Einzelfaser, insbesondere nach 60 Tagen bei 80°C und < 5 % relativer Luftfeuchte in einem Elutionstest weniger als $5000*10^{-7}$ mg pro Einzelfaser beträgt.

In einer weiteren Ausführungsform gemäß dem zweiten Aspekt ist die Hohlfasermembran dadurch gekennzeichnet, dass auf wenigstens einer Oberfläche der Hohlfasermembran, insbesondere auf dem Lumen der Hohlfasermembran, zusätzlich Polyvinylpyrrolidon aufgebracht ist.

In einer weiteren Ausführungsform gemäß dem zweiten Aspekt ist die Hohlfasermembran dadurch gekennzeichnet, dass die Hohlfasermembran auf der lumenseitigen Oberfläche ein Zeta-Potential von -1 mV bis weniger als -7 mV, insbesondere -1 mV bis -4 mV aufweist.

In einer weiteren Ausführungsform gemäß dem zweiten Aspekt ist die Hohlfasermembran dadurch gekennzeichnet, dass die Konzentration an Polyvinylpyrrolidon in einer oberflächennahen Schicht der wenigstens einen hydrophilen Oberfläche der Hohlfasermembran nach XPS Messung 22 % oder mehr, insbesondere 24 bis 34 %, weiter insbesondere 26 bis 34 % beträgt.

In einer weiteren Ausführungsform gemäß dem zweiten Aspekt ist die Hohlfasermembran dadurch gekennzeichnet, dass das Peakhöhenverhältnis von $CNO^-$ und $SO_2^-$, bestimmt mittels TOF-SIMS, an der Oberflächenschicht des inneren Lumens der Hohlfasermembran 4,5 oder mehr, insbesondere 5,5 oder mehr, weiter insbesondere 6,0 oder mehr beträgt.

In einer weiteren Ausführungsform gemäß dem zweiten Aspekt ist die Hohlfasermembran dadurch gekennzeichnet, dass die Hohlfasermembran einen Gehalt an Polyvinylpyrrolidon von 3 bis 5 Gew.% aufweist.

In einer weiteren Ausführungsform gemäß dem zweiten Aspekt ist die Hohlfasermembran dadurch gekennzeichnet, dass das gewichtsmittlere Molekulargewicht (Mw) des PVP der Oberfläche des Lumens der Membran höher ist als das des PVP des Volumens der Membran.

In einer weiteren Ausführungsform gemäß dem zweiten Aspekt ist die Hohlfasermembran dadurch gekennzeichnet, dass das gewichtsmittlere Molekulargewicht (Mw) des PVP der Oberfläche des Lumens der Membran größer als 1.000.000 g/mol, bevorzugt größer als 2.000.000 g/mol, besonders bevorzugt größer als 1.000.000 g/mol bis 3.000.000 g/mol, weiter bevorzugt größer als 2.000.000 g/mol bis 3.000.000 g/mol ist und das gewichtsmittlere Molekulargewicht (Mw) des PVP des Volumens der Membran kleiner als 1.000.000 g/mol, bevorzugt 500.000 g/mol bis kleiner als 1.000.000 g/mol ist.

In einer weiteren Ausführungsform gemäß dem zweiten Aspekt ist die Hohlfasermembran dadurch gekennzeichnet, dass das Verhältnis der gewichtsmittleren Molekulargewichte des PVP im Koagulationsmittel zu dem gewichtsmittleren Molekulargewicht des PVP in der Spinnmasse mindestens 1,2, bevorzugt mindestens 2, weiter bevorzugt 1,2 bis 3, weiter bevorzugt 2 bis 3 beträgt.

In einer weiteren Ausführungsform gemäß dem zweiten Aspekt ist die Hohlfasermembran dadurch gekennzeichnet, dass das Verhältnis der Siebkoeffizienten für Albumin, gemessen nach 5 min, zu dem Siebkoeffizienten gemessen nach 30 min, gemäß der Messmethode der Beschreibung, weniger als 7, insbesondere weniger als 5 beträgt.

In einer weiteren Ausführungsform gemäß dem zweiten Aspekt ist die Hohlfasermembran dadurch gekennzeichnet, dass das Verhältnis der Siebkoeffizienten für Albumin, gemessen nach 5 min, zu dem Siebkoeffizienten gemessen nach 10 min, gemäß der Messmethode der Beschreibung, weniger als 3, insbesondere weniger als 2 beträgt.

Die Untergrenze für die genannten Verhältnisse der Siebkoeffizienten beträgt jeweils 1.

In einer weiteren Ausführungsform gemäß dem zweiten Aspekt ist die Hohlfasermembran dadurch gekennzeichnet, dass Wasser bei der Benetzung der wenigstens einen hydrophilen Oberfläche der Hohlfasermembran einen Kontaktwinkel, gemessen nach der Methode der Steighöhe des Wassers in Kapillaren, von kleiner 57°, insbesondere kleiner 55°, weiter insbesondere kleiner 47° ausbildet, wobei die Untergrenze für den Kontaktwinkel typischerweise bei weniger als 30°, bevorzugt 25°, weiter bevorzugt 20° liegt. Der Kontaktwinkel wird dabei nach der Methode "Bestimmung des Kontaktwinkels θ", wie in vorliegender Anmeldung beschrieben, bestimmt. Unter der hydrophilen Oberfläche ist diejenige Oberfläche der Hohlfasermembran zu verstehen, die die höhere Hydrophilie aufweist bzw. den geringeren Kontaktwinkel mit Wasser ausbildet. Bevorzugt wird die hydrophile Oberfläche im Lumen der Hohlfasermembran ausgebildet.

Ein dritter Aspekt der Anmeldung betrifft die Verwendung eines Koagulationsmittels enthaltend 0,5 g bis 4 g eines hydrophilen Polymers pro kg Koagulationsmittel, insbesondere eines Polyvinylpyrrolidons, in einem Verfahren zur Herstellung einer Hohlfasermembran aufweisend ein Membranmaterial enthaltend ein hydrophobes und ein hydrophiles Polymer, insbesondere Polysulfon und Polyvinylpyrrolidon, und wenigstens ein nicht wasserlösliches Antioxidans, insbesondere ein fettlösliches Vitamin, weiter insbesondere ein α-Tocopherol oder Tocotrienol, zur Hydrophilisierung und Biokompatibilisierung der durch das Verfahren hergestellten Hohlfasermembran.

In einem vierten Aspekt betrifft die Anmeldung einen Hohlfasermembranfilter aufweisend eine Vielzahl von Hohlfasermembranen nach einer Ausführungsform gemäß dem zweiten Aspekt der Erfindung oder von Hohlfasermembranen, die nach einem Verfahren gemäß einer Ausführungsform des ersten Aspekts der Anmeldung hergestellt wurden.

In einem fünften Aspekt betrifft die Anmeldung einen Dialysator für die Hämodialyse, aufweisend eine Vielzahl von Hohlfasermembranen gemäß des zweiten, sechsten, siebten, achten oder neunten Aspektes der Erfindung oder hergestellt nach einem Verfahren des ersten Aspektes der Erfindung.

In einem sechsten Aspekt betrifft die Anmeldung eine Hohlfasermembran, aufweisend ein Membranmaterial, das ein hydrophobes und ein hydrophiles Polymer umfasst, dadurch gekennzeichnet, dass die Elution des hydrophilen Polymers, insbesondere von Polyvinylpyrrolidon, nach einem Lagerzeitraum von 30 Tagen bei 80°C und einer relativen Luftfeuchte von < 5 %, weniger als $4000*10^{-7}$ mg/ pro Einzelfaser, insbesondere nach 60 Tagen bei 80°C und einer relativen Luftfeuchte von < 5 % weniger als $5000*10^{-7}$ mg / Einzelfaser beträgt und dass die Hohlfasermembran auf der lumenseitigen Oberfläche ein Zeta-Potential von -1 mV bis weniger als -7 mV, insbesondere -1 mV bis -5 mV, weiter insbesondere -1 bis -4 mV, aufweist.

In einem siebten Aspekt betrifft die Anmeldung eine Hohlfasermembran, aufweisend ein Membranmaterial, das ein hydrophobes und ein hydrophiles Polymer umfasst, dadurch gekennzeichnet, dass die Elution des hydrophilen Polymers, insbesondere von Polyvinylpyrrolidon, nach einem Lagerzeitraum von 30 Tagen bei 80°C und einer relativen Luftfeuchte von < 5 %, weniger als $4000*10^{-7}$ mg/ pro Einzelfaser, insbesondere nach 60 Tagen bei 80°C und einer relativen Luftfeuchte von < 5 % weniger als $5000*10^{-7}$ mg / Einzelfaser beträgt und die Oberfläche des inneren Lumens einen Kontaktwinkel mit Wasser, gemessen nach der Methode **"Bestimmung des Kontaktwinkels θ"** von weniger als 57°, insbesondere weniger als 55°, weiter insbesondere weniger als 47° aufweist, wobei die Untergrenze für den Kontaktwinkel typischerweise bei weniger als 30°, bevorzugt 25°, weiter bevorzugt 20° liegt.

In einem achten Aspekt betrifft die Anmeldung eine Hohlfasermembran, aufweisend ein Membranmaterial, das ein hydrophobes und ein hydrophiles Polymer umfasst, dadurch gekennzeichnet, dass die Elution des hydrophilen Polymers, insbesondere von Polyvinylpyrrolidon, nach einem Lagerzeitraum von 30 Tagen bei 80°C und einer relativen Luftfeuchte von < 5 %, weniger als $4000*10^{-7}$ mg/ pro Einzelfaser, insbesondere nach 60 Tagen bei 80°C und einer relativen Luftfeuchte von < 5 % weniger als $5000*10^{-7}$ mg / Einzelfaser beträgt, wobei das Verhältnis der Siebkoeffizienten für Albumin, gemessen nach 5 min, zu dem Siebkoeffizienten gemessen nach 30 min, gemäß der Messmethode der Beschreibung, weniger als 7, insbesondere weniger als 5 beträgt.

In einem neunten Aspekt betrifft die Anmeldung eine Hohlfasermembran, aufweisend ein Membranmaterial, das ein hydrophobes und ein hydrophiles Polymer umfasst, dadurch gekennzeichnet, dass die Elution des hydrophilen Polymers, insbesondere von Polyvinylpyrrolidon, nach einem Lagerzeitraum von 30 Tagen bei 80°C und einer relativen Luftfeuchte von < 5 %, weniger als $4000*10^{-7}$ mg/ pro Einzelfaser, insbesondere nach 60 Tagen bei 80°C und einer relativen Luftfeuchte von < 5 % weniger als $5000*10^{-7}$ mg / Einzelfaser beträgt, wobei das Verhältnis der Siebkoeffizienten für Albumin, gemessen nach 5 min, zu dem Siebkoeffizienten gemessen nach 10 min, gemäß der Messmethode der Beschreibung, weniger als 3, insbesondere weniger als 2 beträgt.

Die Untergrenze für die genannten Verhältnisse der Siebkoeffizienten beträgt jeweils 1.

In einem zehnten Aspekt betrifft die Anmeldung eine Hohlfasermembran, aufweisend ein Membranmaterial, das ein hydrophobes und ein hydrophiles Polymer umfasset, dadurch gekennzeichnet, dass der Plättchenverlust, gemessen nach der Methode "Bestimmung des "Platelet Loss" (Absolutmethode)", weniger als 50 %, bevorzugt weniger als 30 %, besonders bevorzugt weniger als 20 % beträgt. Dabei ist es mit der erfindungsgemäßen Hohlfasermembran möglich, Plättchenverluste von Null oder minimal darüber, z.B. im Bereich 10 % oder weniger, z.B. 5 %, 3 % oder 1 %, zu realisieren.

**Detaillierte Beschreibung der Erfindung**

Ein **erster Aspekt** der vorliegenden Erfindung betrifft ein Verfahren zur Herstellung einer Hohlfasermembran, das die folgenden Schritte aufweist:

- Bereitstellen mindestens einer Spinnmasse aufweisend Polysulfon und Polyvinylpyrrolidon, zumindest ein aprotisches polares Lösungsmittel und ein $\alpha$-Tocopherol, insbesondere ein Tocotrienol,
- Bereitstellen mindestens eines Koagulationsmittels aufweisend zumindest ein aprotisches polares Lösungsmittel, und mindestens ein Nicht-Lösemittel, insbesondere Wasser,
- Fördern der Spinnmasse, insbesondere durch wenigstens einen Ringspalt einer Spinndüse zu einem hohlem Spinnfaden,
- Fördern des Koagulationsmittels, insbesondere durch eine zentrale Bohrung der Spinndüse in das Lumen des Spinnfadens,
- Einleiten des Spinnfadens in ein Fällbad,

dadurch gekennzeichnet, dass die Spinnmasse 0,001 bis 0,05 Gew.% des $\alpha$-Tocopherols, oder insbesondere des Tocotrienols, enthält und das weiterhin das Koagulationsmittel 0,5 bis 4 g Polyvinylpyrrolidon pro kg des Koagulationsmittels enthält.

In einer Ausführungsform des erfindungsgemäßen Verfahrens weist das Koagulationsmittel bis zu 1g/kg, insbesondere bis zu 1,5 g pro kg , insbesondere bis zu 2 g pro kg , insbesondere bis zu 2,5 g pro kg , insbesondere bis zu 3 g pro kg , weiter insbesondere weniger als 4 g pro kg eines hydrophilen Polymers, insbesondere eines Polyvinylpyrrolidons, auf.

Das erfindungsgemäße Verfahren hat den Vorteil, dass das Polyvinylpyrrolidon, das im Koagulationsmittel gelöst vorliegt, während der Herstellung der Hohlfasermembran auf die innere Oberfläche der Hohlfasermembran aufgebracht werden kann. Es hat sich insbesondere gezeigt, dass das Aufbringen eines hydrophilen Polymers auf die innere Oberfläche der Hohlfasermembran zu einer erhöhten Hydrophilisierung und damit zu einer gesteigerten Blutkompatibilisierung der Hohlfasermembran führt. Der erhöhte Anteil des Polyvinylpyrrolidons, der auf die innere Oberfläche der Hohlfasermembran aufgebracht wird, führt zu einer Verringerung der Komplementaktivierung, gemessen als C5a, und einer Verringerung des "Platelet Loss", im Vergleich zu Werten einer Vergleichshohlfasermembran, auf die kein Polyvinylpyrrolidon auf die innere Oberfläche der Hohlfasermembran aufgebracht wurde.

Das erfindungsgemäße Verfahren hat weiterhin den Vorteil, dass das Polyvinylpyrrolidon, das nach dem erfindungsgemäßen Verfahren auf der Oberfläche der Hohlfasermembran aufgebracht wurde, durch den Anteil des nicht wasserlöslichen Antioxidans, insbesondere des fettlöslichen Vitamins, weiter insbesondere des $\alpha$-Tocopherols oder Tocotrienols, in der Hohlfasermembran auf der Oberfläche fixiert werden kann.

Der Begriff "*fixiert*" bedeutet in diesem Zusammenhang, dass das Polyinylpyrrolidon, auf der Oberfläche der Hohlfasermembran in Kontakt mit Flüssigkeiten nur in verringertem Maße eluiert werden kann. Insbesondere reicht es bereits aus, dass das nicht wasserlösliche Antioxidans, insbesondere das fettlösliche Vitamin, in einem Anteil von 0,001 Gew. % in der Spinnmasse vertreten ist, um einen fixierenden Effekt auf das wasserlösliche Polymer, insbesondere das Polyvinylpyrrolidon, das aus dem Koagulationsmittel auf die Oberfläche der Hohlfasermembran abgeschieden wird, zu bewirken. Unterhalb eines Anteils von kleiner 0,001 Gew. % des fettlöslichen Vitamins in der Spinnmasse ist der fixierende Effekt gegenüber dem Polyvinylpyrrolidon, aus dem Koagulationsmittels zu niedrig ausgeprägt, um dadurch signifikante Verbesserungen in der Blutkompatibilisierung feststellen zu können. Oberhalb eines Anteils von 0,05 % an wasserunlöslichem Antioxidans, insbesondere an fettlöslichem Vitamin, in der Spinnmasse nimmt eine Fixierung des durch das im Koagulationsmittel eingebrachte Polyvinylpyrrolidon, nicht mehr nennenswert zu. Zudem kann durch einen höheren Anteil an nicht wasserlöslichem Antioxidans, insbesondere an fettlöslichem Vitamin, in der Spinnmasse der durch Auftragung des Polyvinylpyrrolidons, bewirkte hydrophile Charakter der Oberfläche der Hohlfasermembran herabgesenkt werden. Die Herabsetzung des hydrophilen Charakters kann sich wiederum negativ auf die Blutkompatibilität, insbesondere negativ auf den "Platelet Loss", auswirken.

Die nach dem Verfahren gemäß des ersten Aspekts der Erfindung hergestellten Hohlfasermembranen zeichnen sich insbesondere durch eine Verbesserung der Blutkompatibilität aus. Insbesondere wurde hinsichtlich der Komplementaktivierung, gemessen als C5a, gefunden, dass diese deutlich gegenüber Vergleichshohlfasermembranen bestehend aus einem Polysulfon und Polyvinylpyrrolidonmaterial herabgesetzt ist. Die Komplementaktivierung wird dabei als ein Hämokompatibilitätsmarker angesehen, der für die Bewertung der Biokompatibilität von Hohlfasermembranen herangezogen wird. Insbesondere hat sich gezeigt, dass der Wert für die C5a-Aktivierung gegenüber einer standardmäßigen Hohlfasermembran bestehend aus Polysulfon und Polyvinylpyrrolidon um wenigstens 50 % herabgesenkt ist. Dies bedeutet, dass die Komplementaktivierung der erfindungsgemäß hergestellten Hohlfasermembran lediglich 50 % gegenüber einer handelsüblichen Vergleichshohlfasermembran beträgt. In alternativen Ausführungsformen ist es nach dem erfindungsgemäßen Herstellverfahren gemäß des ersten Aspekts der Erfindung möglich, Hohlfasermembranen herzustellen, die eine C5-Komplementaktivierung von bis 40 %, bevorzugt bis 30 %, bevorzugt bis 20 % bevorzugt bis 10 %, weiter bevorzugt bis nur 8 % der Komplementaktivierung verursachen, die an einer Vergleichshohlfasermembran festgestellt werden kann. Insbesondere ist es durch das erfindungsgemäße Verfahren möglich, durch Zugabe eines α-Tocopherols oder Tocotrienols zur Spinnmasse im Bereichen von 0,001 bis 0,05 Gew.% und der Zugabe von Polyvinylpyrrolidon, zum Koagulationsmittel in Anteilen von 0,5 g bis 4 g pro kg Koagulationsmittel, die Eigenschaften der Komplementaktivierung der Hohlfasermembran einzustellen. Dabei ist die Anwendung des erfindungsgemäßen Verfahrens natürlich nicht ausschließlich auf eine optimiert reduzierte Komplementaktivierung auszurichten, sondern so zu steuern, dass auch weitere Hämokompatibilitätsmarker, insbesondere der "Platelet Loss"-Wert sowie auch die Leistungsparameter der Hohlfasermembran nach Möglichkeit günstige Werte annehmen können.

Unter dem Begriff "*Vergleichshohlfasermembran*" ist im Sinne der vorliegenden Erfindung eine Hohlfasermembran zu verstehen, die unter denselben Spinnbedingungen und mit denselben Anteilen der hydrophoben und hydrophilen Polymeren, insbesondere Polysulfon und Polyvinylpyrrolidon, hergestellt wurde, die aber kein nicht wasserlösliches Antioxidans, insbesondere kein fettlösliches Vitamin, in der Hohlfasermembran aufweist und auch nicht zusätzlich mit einem wasserlöslichen Polymer, insbesondere Polyvinylpyrrolidon, an zumindest einer Oberfläche der Hohlfasermembran beschichtet wurde. Im vorliegenden Fall ist eine derartige Hohlfasermembran durch den handelsüblichen Dialysator "Fresenius FX 60" bereitgestellt.

Unter dem Begriff "*Koagulationsmittel*" im Sinne der vorliegenden Erfindung ist ein Mittel zu verstehen, welches während des Durchlaufens des Spinnfadens durch den Fällspalt die Phaseninversion im Spinnfaden bewirkt und die Porenstruktur der Hohlfasermembran mitbestimmt. Das Koagulationsmittel weist zumindest ein polares aprotisches Lösemittel und ein Nicht-Lösemittel sowie erfindungsgemäß ein wasserlösliches Polymer, insbesondere Polyvinylpyrrolidon, auf.

Das Koagulationsmittel weist vorzugsweise 25 bis 60 Gew.% insbesondere 35 bis 55 %, eines polar aprotischen Lösungsmittels, insbesondere Dimethylacetamid, und 40 bis 75 % Gew.%, insbesondere 45 bis 65 % eines polar protischen Nicht-Lösemittels, insbesondere Wasser, bezogen auf die Gesamtmasse des Koagulationsmittels auf. Zudem weist das Koagulationsmittel 0,5 g bis 4 g pro kg Koagulationsmittel, insbesondere mehr als 1 g, insbesondere mehr als 1,5 g, insbesondere mehr als 2 g, insbesondere mehr als 2,5 g, insbesondere weniger als 4 g insbesondere weniger als 3 g pro kg Koagulationsmittel Polyvinylpyrrolidon, auf.

Die Bezeichnung "*Lösemittel*" und "*Nicht-Lösemittel*" bezieht sich im Rahmen der vorliegenden Anmeldung auf die Lösungseigenschaften gegenüber dem membranbildenden hydrophoben Polymer, das gemäß dem erfindungsgemäßen Verfahren Hauptbestandteil der hergestellten Hohlfasermembran ist. Demgemäß gelten polar aprotische Flüssigkeiten, wie DMAc (Dimethylacetamid), DMF (Dimethylformamid), DMSO (Dimethylsulfoxid), NMP (N-Methylpyrrolidon) als Lösungsmittel, da sich das membranbildende Polymer in diesen Flüssigkeiten oder ihren Mischungen lösen lässt. Dagegen gelten im Rahmen dieser Anmeldung polar protische Flüssigkeiten, wie z.B. Wasser, Ethanol, Essigsäure als Nicht-Lösemittel, da sie das membranbildende Polymer nicht lösen und daher dazu verwendet werden können, die Spinnmasse in der Herstellung von Hohlfasermembranen zur Fällung zu bringen.

In einer Ausführungsform ist das erfindungsgemäße Verfahren dadurch gekennzeichnet, dass das im Koagulationsmittel enthaltene Polyvinylpyrrolidon, eine Molekulargewichtsverteilung im Bereich von 200.000 g/mol bis 2.000.000 g/mol aufweist, insbesondere das gewichtsmittlere Molekulargewicht 900.000 g/mol beträgt.

Das Aufbringen des Polyvinylpyrrolidons, gemäß des erfindungsgemäßen Herstellverfahrens aus dem Koagulationsmittel auf wenigstens eine Oberfläche, insbesondere die innere Oberfläche der Hohlfasermembran, wird durch das Molekulargewicht des eingesetzten hydrophilen Polymers beeinflusst. Hydrophile Polymere, insbesondere Polyvinylpyrrolidon, mit niedrigem Molekulargewicht neigen weniger dazu, auf der Oberfläche des Spinnfadens bzw. Hohlfasermembran, fixiert zu werden. Hydrophile Polymere, insbesondere Polyvinylpyrrolidonmoleküle, mit höherem Molekulargewicht zeigen dagegen stärkere Adsorptionseigenschaften auf und werden daher besser auf der inneren Oberfläche des Spinnfadens oder der Hohlfasermembran fixiert. Die Verwendung von hydrophilen Polymers, insbesondere Polyvinylpyrrolidon, deren Molekulargewichtsverteilung in einem Bereich von 200.000 g/mol bis 2.000.000 g/mol liegt, hat sich dementsprechend als vorteilhaft herausgestellt. In einer normalerweise gaußförmigen Molekulargewichtsverteilung des hydrophilen Polymeren, insbesondere des Polyvinylpyrrolidons, sind die Anteile von niedrig molekularen Polymeren schwächer konzentriert als die Polymere im mittleren Molekulargewichtsbereich. Als ein Beispiel für ein geeignetes hydrophiles Polymer sei hier ein handelsübliches Polyvinylpyrrolidon mit der Bezeichnung K80 bis K90 genannt.

Insbesondere eignet sich nach dem erfindungsgemäßen Herstellverfahren ein Polyvinylpyrrolidon, mit einem gewichtsmittleren Molekulargewicht von 700.000 g/mol bis 1.200.000 g/mol, insbesondere 900.000 g/mol und/oder einer Molekulargewichtsverteilung im Bereich von 200.000 bis 2.000.000 g/mol bevorzugt für das Aufbringen von hydrophilem Polymer, insbesondere von Polyvinylpyrrolidon, auf die innere Oberfläche des Spinnfadens oder der Hohlfasermembran.

In einer weiteren Ausführungsform ist das erfindungsgemäße Verfahren dadurch gekennzeichnet, dass das hydrophile Polymer der Spinnmasse Polyvinylpyrrolidon (PVP) umfasst und dass das hydrophile Polymer im Koagulationsmittel Polyvinylpyrrolidon (PVP) umfasst, wobei das gewichtsmittlere Molekulargewicht (Mw) des PVP im Koagulationsmittel höher ist als das des PVP in der Spinnmasse. Es hat sich gezeigt, dass es sehr vorteilhaft ist, wenn das PVP, welches in das Koagulationsmittel eingebracht wird, ein möglichst hohes gewichtsmittleres Molekulargewicht aufweist, da die Verwendung eines solchen PVP-Typs zu einer sehr hohen Bedeckung der Lumenoberfläche mit PVP führt. Nachteiligerweise erhöht sich jedoch die Viskosität einer Lösung durch die Erhöhung des Molekulargewichtes eines Polymers bei gleicher Konzentration mit dem Molekulargewicht. Durch die erfindungsgemäße Verwendung derart geeigneter PVP-Typen mit optimal eingestellten Molekulargewichten kann insbesondere die Viskosität der Spinnmasse in einem optimal niedrigen Bereich von weniger als 15.000 mPas, insbesondere von weniger als 5000 mPas, gemessen bei 40°C mit einem Viskosimeter VT550 der Fa. Haake, Deutschland bei der Stufe r.3 (30 U/min) mit einem Drehkörper "MV1 (MV-DIN)" der Fa. Haake (Schergeschwindigkeit 38,7/s), gehalten werden und die Bedeckung des Lumens der Membran durch das höhere gewichtsmittlere Molekulargewicht in dem Koagulationsmittel verbessert werden. Dies führt zu optimalen Hämokompatibilitätswerten und geringen Elutionswerten von PVP nach einem Alterungsversuch. Verarbeitungtechnisch ist es erforderlich, eine minimale Spinnmassenviskosität, gemessen nach der oben beschriebenen Methode, von mindestens 800 mPas einzuhalten.

Bei einem weiteren ausgeführten Verfahren beträgt das gewichtsmittlere Molekulargewicht (Mw) des PVP in der Spinnmasse unter 1.000.000 g/mol, z.B. 995.000 g/mol oder 900.000 g/mol, bevorzugt 500.000 g/mol bis unter 1.000.000 g/mol, und das gewichtsmittlere Molekolargewicht (Mw) des PVP in dem Koagulationsmittel über 1.000.000 g/mol, z.B. 1.005.000 g/mol oder 1.100.000 g/mol, insbesondere über 2.000.000 g/mol, bevorzugt größer als 1.000.000 bis 3.000.000 g/mol, weiter bevorzugt größer als 2.000.000 g/mol bis 3.000.000 g/mol. Bevorzugt beträgt das Verhältnis der gewichtsmittleren Molekulargewichte des PVP im Koagulationsmittel zu dem gewichtsmittleren Molekulargewicht des PVP in der Spinnmasse mindestens 1,2, bevorzugt mindestens 2, weiter bevorzugt 1,2 bis 3, weiter bevorzugt 2 bis 3. Dies kann beispielsweise dadurch bewerkstelligt werden, dass als PVP ein PVP K81/86 der Fa. Ashland für die Zugabe zur Spinnmasse Verwendung findet und als PVP für die Zugabe zum Koagulationsmittel ein PVP K90 oder besonders bevorzugt K120 Verwendung findet. Solche Ausführungsformen des Verfahrens führen zu besonders gut und leicht ausführbaren Herstellungsprozessen mit einer optimalen Spinnmasseviskosität und somit zu einem optimalen asymmetrischen Membranaufbau mit guter Siebkurve und gleichzeitig zu einer besonders hämokompatiblen Membran mit niedrigen Elutionswerten für PVP nach Alterung und guter Blutverträglichkeit.

Der Begriff "*Molekulargewichtsverteilung*" ist aus der Polymerphysik bekannt und definiert. Im Sinne der vorliegenden Anmeldung wird unter einer Molekulargewichtsverteilung einer Polymerprobe die Wahrscheinlichkeitsdichteverteilung verstanden, mit der ein Polymermolekül mit einem bestimmten Molekulargewicht in der Polymerprobe anzutreffen ist. Die Molekulargewichtsverteilung von Polyvinylpyrrolidonen oder Polyethylenglykolen kann durch bekannte Messmethoden festgestellt werden, wie beispielsweise durch Gelpermeationschromatographie (GPC), gekoppelt mit einem geeigneten Lichtstreudetektor, z.B. einem MultiAngleLaserLightscattering (MALLS) Detektor.

Der Begriff "*gewichtsmittleres Molekulargewicht*, *(Mw)*" gibt das Molekulargewicht an, dessen Gewichtsanteil in einer Polymerprobe am häufigsten aufzufinden ist. Bei einer bekannten Molekulargewichtsverteilung gibt das gewichtsmittlere Molekulargewicht einen charakteristischen Mittelwert für eine Polymerprobe, im vorliegenden Fall für ein Polyvinylpyrrolidon oder Polyethylenglykol, wider, von dem der Fachmann auf die vorliegende Molekülgröße der Polymere in der Polymerprobe schließen kann.

In einer Ausführungsform des erfindungsgemäßen Herstellverfahrens wird die Ringspaltdüse auf eine Temperatur von 30 bis 85°C, insbesondere 65 bis 85°C, temperiert.

Durch Temperierung der Ringspaltdüse werden Spinnmasse und Koagulationsmittel im Inneren des Spinnfadens beim Fördern auf dieselbe Temperatur oder nahezu dieselbe Temperatur gebracht. Durch Einstellung der Temperatur der extrudierten Spinnmasse und des extrudierten Koagulationsmittels kann der Prozess der Koagulation, während der Spinnfaden den Fällspalt durchläuft, beeinflusst werden. Insbesondere ist die Temperatur der Ringspaltdüse aber so voreinzustellen, dass auch eine angestrebte Porenstruktur der Hohlfasermembran gebildet wird.

Die Abzugsgeschwindigkeit des Spinnfadens beim erfindungsgemäßen Herstellverfahren beträgt 100 mm/s bis 1500 mm/s. Die Abzugsgeschwindigkeit des Spinnfadens ist bei vorgegebener Höhe des Fällspalts mitbestimmend für die Durchlaufzeit des Spinnfadens.

Der Begriff "*Fällspalt*" bezeichnet dabei den Abstand zwischen der Spinndüse und dem Flüssigkeitsniveau des Fällbades.

Der Begriff "*Durchlaufzeit*" bezeichnet die Zeitdauer, die die Spinnmasse benötigt, um den Fällspalt von der Spinndüse bis zum Flüssigkeitsniveau des Fällbades zu durchlaufen. Mit Hilfe der Durchlaufzeit kann insbesondere die Porenstruktur der Außenseite beeinflusst werden.

In einem **zweiten Aspekt** betrifft die Erfindung eine Hohlfasermembran mit verbesserter Biokompatibilität, die aus einem Membranmaterial besteht, welches Polysulfon, Polyvinylpyrrolidon und $\alpha$-Tocopherol, insbesondere Tocotrienol aufweist, wobei das $\alpha$-Tocopherol oder Tocotrienol in einem Anteil von 0,005 bis 0,25 Gew.% bezogen auf das Gesamtgewicht des Membranmaterials vorhanden ist. Ein weiteres Beispiel für ein nicht wasserlösliches Antioxidans ist Pentaerythritol tetrakis (3,5-di- *tert*-butyl-4- hydroxyhydrocinnamate), (Irganox 1010, Fa. BASF).

Derartige Hohlfasermembranen eignen sich daher besonders für die extrakorporale Blutbehandlung, bei der Patientenblut mit dem Membranmaterial der Hohlfasermembranen in Kontakt kommt. Insbesondere eignen sich daher die erfindungsgemäßen Hohlfasermembranen für den Bau von Hohlfasermembranfiltern für die extrakorporale Blutbehandlung, da sie überwiegend in Therapien von nierengeschädigten Patienten zum Einsatz kommen. Die verringerte Elution von Polyvinylpyrrolidon, erweist sich dabei für den Patienten als gesundheitlicher Vorteil, da eluierbares hydrophiles Polymer über den extrakorporalen Blutkreislauf in den Organismus des Patienten gelangen kann.

Darüber hinaus wird durch die verringerte Elution von Polyvinylpyrrolidon, auch über den Zeitraum der extrakorporalen Blutbehandlung eine konstant bessere Hydrophilie der Hohlfasermembran gewährleistet. Die verbesserte Hydrophilie bedingt eine bessere Benetzbarkeit der Hohlfasermembran mit Blut. Damit einher geht eine verbesserte Blutkompatibilität, die an einer geringeren Komplementaktivierung (C5a) erkannt werden kann. Die Hydrophilie verursacht zudem einen verringerten "Platelet Loss", da die Thrombozyten an hydrophilen Oberflächen weniger anhaften und damit der Beginn der Blutgerinnungskaskade verhindert wird.

Der Begriff "*nicht wasserlösliches Antioxidans*" bezeichnet einen Stoff mit oxidationsverhindernder Wirkung mit einer Wasserlöslichkeit von weniger als 2 mg/l bei einer Temperatur von 25°C. Der Begriff "*fettlösliches Vitamin*" bezeichnet im Sinne der vorliegenden Anmeldung ein Vitamin, das sich im Fettgewebe des menschlichen Organismus anreichert. Der Begriff ist in Bezug auf die menschliche Physiologie bekannt und bezeichnet damit eine bestimmte Klasse von Vitaminen. Bezogen auf die vorliegende Anmeldung bezieht sich der Begriff "*fettlöslich*" darauf, dass es sich bei dem Vitamin um eine unpolare Substanz handelt, die kaum bzw. nicht wasserlöslich ist. Bezüglich der fettlöslichen Vitamine bildet die Substanzklasse von Vitamin E den bekanntesten Teil der sog. fettlöslichen Vitamine. Vitamin E ist dabei ein Sammelbegriff für fettlösliche Substanzen mit antioxidativer Wirkung. Zu den häufigsten Vertretern des Vitamin E gehören $\alpha$-Tocopherol und Tocotrienol.

Die erfindungsgemäße Hohlfasermembran weist Polysulfon als hydrophobes Polymer auf. Unter "*Polysulfon*" wird im Sinne der vorliegenden Anmeldung ein Polymer verstanden, das eine Sulfongruppe in der Hauptkette des Polymers aufweist. Typische Vertreter von Polysulfonen sind Polysulfon auf der Basis von Bisphenol-A (PSU) oder Polyethersulfon (PES). Weitere Vertreter von Polysulfonpolymeren sind im Stand der Technik bekannt und im Sinne der vorliegenden Anmeldung für die Herstellung von Blutbehandlungsmembranen geeignet. Polysulfonpolymere haben sich in der Herstellung von Blutbehandlungsmembranen gegenüber anderen Polymeren als überlegen erwiesen, da sie dampfsterilisierbar sind und gute Eigenschaften hinsichtlich der Hämokompatibilität aufweisen. Der Gewichtsanteil des hydrophoben Polymers in der Hohlfasermembran beträgt 94 bis 97,5 %.

Weiterhin weist die erfindungsgemäße Hohlfasermembran als hydrophiles Polymer Polyvinylpyrrolidon auf. Unter "*Polyvinylpyrrolidon*" wird ein Polymer verstanden, das unter Verwendung des Monomeren Vinylpyrrolidon oder Derivaten davon hergestellt wird. Insbesondere eignet sich "*Polvinylpyrrolidon*" (auch als PVP bezeichnet) im Sinne der vorliegenden Erfindung für die Herstellung erfindungsgemäßer Hohlfasermembranen. Polyvinylpyrrolidon ist ein wasserlösliches, hydrophiles Polymer, das in der Produktion von Hohlfasermembranen auf Basis von Polysulfon verwendet wird. Zudem bewirkt Polyvinylpyrrolidon eine Verbesserung der Hämokompatibilität von Hohlfasermembranen, die hydrophobe Polymere umfassen, da hydrophobe Hohlfasermembranen hydrophilisiert und dadurch besser für Blut benetzbar werden. Der Gewichtsanteil des hydrophilen Polymers in der Hohlfasermembran beträgt 3 - 5 %.

Unter "*Hämokompatibilität*" wird im Sinne der vorliegenden Anmeldung dabei die Verträglichkeit zu Humanblut verstanden, insbesondere wird darunter verstanden, dass Blut in Kontakt mit den Materialien der Hohlfasermembran keine negativen Reaktionen eingeht, die für den Patienten im Rahmen einer Blutbehandlungstherapie gesundheitsschädlich sein können. Beispielhaft können hierunter Aktivierungsprozesse des Komplementsystems, Blutgerinnungssystems, Kontaktphasensystems sowie korpuskulärer Bestandteile des Blutes verstanden werden. Die Verwendung von Polysulfon-/Polyvinylpyrrolidon-Polymeren haben sich gegenüber anderen Blutkontaktmaterialien in Hohlfasermembranen hinsichtlich ihrer Blutkompatibilität als überlegen erwiesen.

Die erfindungsgemäße Hohlfasermembran zeichnet sich in einer alternativen Ausführung gemäß dem zweiten Aspekt der Erfindung dadurch aus, dass die Hohlfasermembran auf zumindest einer Oberfläche zumindest teilweise zusätzlich mit einem hydrophilen Polymer, insbesondere Polyvinylpyrrolidon, beschichtet ist.

Die Beschichtung mit Polyvinylpyrrolidon, verursacht eine weitere Hydrophilisierung der Hohlfasermembran auf der wenigstens einen beschichteten Oberfläche der Hohlfasermembran. Es hat sich gezeigt, dass die Hohlfasermembran nach dem zweiten Aspekt der Erfindung durch den geringen Gehalt an $\alpha$-Tocopherol oder Tocotrienol, eine Zurückhaltung von hydrophilem Polymer gegenüber anströmenden wässrigen Flüssigkeiten, z.B. Blut oder Wasser, verursacht. Es wurde weiter beobachtet, dass Polvinylpyrrolidon, das durch einen Beschichtungsprozess auf die wenigstens eine Oberfläche der Hohlfasermembran aufgebracht wird, durch das in der Hohlfasermembran enthaltene nicht wasserlösliche Antioxidans, insbesondere das fettlösliche Vitamin, weitgehend fixiert werden kann. Elutionstests ergaben diesbezüglich eine reduzierte Elution von Polyvinylpyrrolidon, gegenüber entsprechend beschichteten Hohlfasermembranen, die kein nicht wasserlösliches Antioxidans im Membranmaterial enthielten. Dabei kann insbesondere die Beschichtung mit Polyvinylpyrrolidon so ausgeführt werden, dass vorzugsweise nur solche Mengen an Polyvinylpyrrolidon auf der wenigstens einen Oberfläche der Hohlfasermembran abgeschieden werden können, die gerade ausreichend sind, um die chemisch hydrophobe polymere Membranoberfläche durch Polyvinylpyrrolidon zu hydrophilisieren.

Die Beschichtung von Polyvinylpyrrolidon, auf wenigstens eine Oberfläche der Hohlfasermembran kann durch eine Beschichtungslösung erfolgen, die das hydrophile Polymer enthält und die auf eine Oberfläche der Hohlfasermembran aufgebracht wird. Es hat sich als vorteilhaft erwiesen, die Beschichtung der Hohlfasermembran mit Polyvinylpyrrolidon, während der Herstellung der Hohlfasermembranen aus hydrophobem und hydrophilen Polymer im Spinnprozess aufzubringen. Dazu wird dem Koagulationsmittel Polyvinylpyrrolidon, zugegeben und zusammen mit der Spinnmasse, aufweisend und Polyvinylpyrrolidon, sowie zumindest ein ein fettlösliches Vitamin, durch die konzentrische Ringspaltdüse gefördert. Der Spinnfaden wird dabei auf der Innenseite von dem im Koagulationsmittel gelösten Polyvinylpyrrolidon, benetzt. Durch den Kontakt mit dem Koagulationsmittel sowie dem Einleiten des Spinnfadens in das Fällbad wird die Membranstruktur ausgebildet und damit auch das Polyvinylpyrrolidon, aus dem Koagulationsmittel auf der Membranoberfläche fixiert.

In einer weiteren Ausführung gemäß des zweiten Aspekts der Erfindung ist die erfindungsgemäße Hohlfasermembran dadurch gekennzeichnet, dass die Hohlfasermembran auf der wenigstens einen hydrophilen Oberfläche, insbesondere der lumenseitigen Oberfläche, ein Zeta-Potential von -1 mV bis -7 mV, insbesondere -1 mV bis -5 mV, insbesondere -1 mV bis -4 mV, aufweist.

Eine Hohlfasermembran, die nach der vorbeschriebenen Art auf zumindest einer Oberfläche der Hohlfasermembran mit Polyvinylpyrrolidon, beschichtet ist, weist ein neutraleres Zeta-Potential auf als eine Vergleichsmembran, die aus denselben Membranmaterialien besteht, aber nicht zusätzlich mit hydrophilem Polymer beschichtet wurde.

Weiterhin weist die erfindungsgemäße Hohlfasermembran bei Benetzung einen minimierten Kontaktwinkel gegenüber Wasser auf. Bei Benetzung einer Oberfläche der erfindungsgemäßen Hohlfasermembran mit Wasser ist der Kontaktwinkel, gemessen nach der Methode der Steighöhe des Wassers in Kapillaren, kleiner 57°, insbesondere kleiner 55°, weiter insbesondere kleiner 47°. Bevorzugt weist die Oberfläche des inneren Lumens einen solch geringen Kontaktwinkel auf.

Der Kontaktwinkel, den Wasser auf einer Membranoberfläche einnimmt, ist ein Maß für die Hydrophilie der Membranoberfläche. Durch die Beschichtung mit Polyvinylpyrrolidon, wird, wie vorab beschrieben, die Membranoberfläche hydrophilisiert, ohne dass der Gesamtgehalt an hydrophilem Polymer der Hohlfasermembran wesentlich ansteigt. Die Beschichtung stellt damit eine insgesamt kostengünstige und prozesstechnisch günstige Lösung dar, um die Hohlfasermembran auf wenigstens einer Membranoberfläche, insbesondere der blutseitigen Oberfläche, zu hydrophilisieren. Insbesondere die Beschichtung mit Polyvinylpyrrolidon verändert den PVP-Gesamtgehalt der Hohlfasermembran kaum, da die Auftragsstärken von PVP minimal sind. Ein geringer Kontaktwinkel steht dabei für eine hohe Hydrophilie der Membraninnenoberfläche. Im Vergleich wurden an handelsüblichen Hohlfasermembranen, z.B. aus dem Dialysator FX 60 der Firma Fresenius, ein Kontaktwinkel von 64° festgestellt. Die FX60 Hohlfasermembranen, umfassend Polysulfon und Polyvinylpyrrolidon, enthalten dabei kein zusätzliches Polyvinylpyrrolidon, welches durch Beschichtung auf die Oberfläche der Membran aufgebracht wurde. Ebenso enthält diese Hohlfasermembran auch keinen Anteil von $\alpha$-Tocopherol im Membranmaterial. Demgegenüber wurde für erfindungsgemäße Hohlfasermembranen, die mit einer Polyvinylpyrrolidon-Konzentration von 1500 ppm und einem $\alpha$-Tocopherol-Gehalt von 0,05 Gew.% in dem Koagulationsmittel hergestellt wurden, ein Kontaktwinkel von 52° festgestellt. Es wurde weiter festgestellt, dass die Verringerung des Kontaktwinkels mit einer Verringerung der Komplementaktivierung, gemessen als C5a, korreliert. Weiterhin wurde festgestellt, dass ein verringerter Kontaktwinkel auch mit einer Verringerung des "Platelet Loss" korreliert.

Die erfindungsgemäße Hohlfasermembran ist weiterhin dadurch gekennzeichnet, dass die Hohlfasermembran eine Komplementaktivierung aufweist, die geringer ist, insbesondere bis zu 50 % geringer ist als die einer Vergleichsmembran, die kein nicht wasserlösliches Antioxidans, insbesondere fettlösliches Vitamin im Membranmaterial und keine Beschichtung mit hydrophilem Polymer, insbesondere PVP, auf mindestens einer Oberfläche der Hohlfasermembran aufweist.

Es hat sich gezeigt, dass sich die Komplementaktivierung durch die Menge an Polyvinylpyrrolidon, das erfindungsgemäß durch das Koagulationsmittel in der Herstellung der Hohlfasermembran auf eine Oberfläche der Hohlfasermembran aufgetragen wird, regulieren lässt. Die Konzentrationen von PVP, in dem Koagulationsmittel sind von 0,5 g bis 4 g, insbesondere bis zu 3 g, insbesondere bis zu 2 g, insbesondere bis zu 1,5 g pro kg Koagulationsmittel.

Weiterhin weist die erfindungsgemäße Hohlfasermembran einen geringeren "Platelet Loss" auf, insbesondere von 60 % gegenüber einer Vergleichshohlfasermembran bestehend aus Polysulfon und Polyvinylpyrrolidon, die kein nicht wasserlösliches Antioxidans im Membranmaterial und keine Polyvinylpyrrolidon-Beschichtung auf zumindest einer Oberfläche der Hohlfasermembran aufweist.

Es hat sich gezeigt, dass die Verringerung der Thrombozytenzahl durch die Menge an aufgetragenem Polyvinylpyrrolidon, und der Menge an fettlöslichem Vitamin, das in der Hohlfasermembran vorhanden ist, gesteuert werden kann. Insbesondere ergibt sich aus der Zusammenwirkung von aufgetragenem Polyvinylpyrrolidon, und $\alpha$-Tocopherol oder Tocotrienol, eine Verringerung des Verlustes an Thrombozyten, der geringer ist als bei einer entsprechenden Vergleichshohlfasermembran.

Nach einer Weiterbildung der Erfindung weist die Hohlfasermembran auf der lumenseitigen Oberfläche eine Konzentration an Polyvinylpyrrolidon in einer oberflächennahen Schicht der Hohlfasermembran auf, die nach XPS-Messung 22 % oder mehr, insbesondere 24 bis 34 %, weiter insbesondere 26 bis 34 % beträgt. Die Analyse wird nach der "Messmethode zur Bestimmung des Polyvinylpyrrolidon in einer oberflächennahen Schicht (XPS)", wie in vorliegender Anmeldung beschrieben, durchgeführt. Die Analyse erfasst dabei oberflächennahe Schichten bis zu einer Tiefe von ca. 10 nm. Solche Membranen weisen eine besonders gute Belegung der Lumenseite mit PVP auf. Dies führt zu einer guten Hydrophilie und somit hohen Biokompatibilität.

Die erfindungsgemäße Hohlfasermembran weist auf wenigstens einer Oberfläche insbesondere auf der hydrophilen Oberfläche, weiter insbesondere auf der lumenseitigen Oberfläche, ein Peakhöhenverhältnis von $CNO^-$ zu $SO_2^-$, bestimmt nach der Methode "Bestimmung des Peakhöhenverhältnisses von $CNO^-$ und $SO_2^-$ mittels TOF-SIMS in einer Oberflächenschicht", wie in vorliegender Anmeldung beschrieben, an der Oberflächenschicht von 4,5 oder mehr, insbesondere von 5,5 oder mehr, weiter insbesondere von 6,0 oder mehr auf. Die Messmethode TOF-SIMS stellt eine Analysenmethode dar, die eine besonders hohe Oberflächenempfindlichkeit aufweist, d.h. dass nur die äußerste Monolage der Oberfläche analysiert wird. Somit kann die Oberflächenbelegung des Lumens der Membran mit dem hydrophilen Polymer besonders gut und sicher bestimmt werden. Dabei ist eine hohe Belegung anzustreben, da so die Hydrophilie und die Biokompatibilität erhöht werden kann.

Durch die hohe Konzentration des Polyvinylpyrrolidons auf der Oberfläche, die im Kontakt mit Behandlungsblut steht, wird die Oberfläche des Polysulfons, maskiert und tritt dadurch nicht mehr mit Blutzellen oder Plasmaproteinen in Kontakt. Die Beschichtung zumindest einer Oberfläche der Hohlfasermembran, die mit Blut in Kontakt kommt, hat den Vorteil, dass mit geringen Mengen von Polyvinylpyrrolidon, bereits eine effektive Beschichtung ermöglicht werden kann und mit dem nicht wasserlöslichen Antioxidans in der Spinnmasse an der Oberfläche der Hohlfasermembran fixiert werden kann. Dadurch wird insbesondere eine kostengünstige Bereitstellung der Hohlfasermembran mit verbesserter Biokompatibilität gewährleistet, da nur geringe Mengen an wasserlöslichem Antioxidans, insbesondere fettlöslichem Vitamin und Polyvinylpyrrolidon, für den Effekt der Biokompatibilisierung eingesetzt werden müssen.

Die erfindungsgemäße Hohlfasermembran weist weiterhin in einem Elutionstestverfahren eine Elution von Polyvinylpyrrolidon, bestimmt nach der *"Messmethode zur Bestimmung der Elution von Polyvinylpyrrolidon*", nach einem Lagerzeitraum von 30 Tagen bei 80°C und < 5 % relativer Luftfeuchte von weniger als $4000*10^{-7}$ mg pro Einzelfaser, insbesondere nach 60 Tagen unter gleichen Lagerbedingungen eine Elution von weniger als $5000*10^{-7}$ mg pro Einzelfaser auf. Bevorzugt weist die Elution von Polyvinylpyrrolidon nach einem Lagerzeitraum von 30 Tagen bei 80°C und < 5 % relativer Luftfeuchte von weniger als $2000*10^{-7}$ mg pro Einzelfaser, insbesondere nach 60 Tagen unter gleichen Lagerbedingungen eine Elution von weniger als $3000*10^{-7}$ mg pro Einzelfaser auf.

Unter der "*Elution von Polyvinylpyrrolidon*" wird in diesem Zusammenhang das Auswaschen von Polyvinylpyrrolidon aus der Hohlfasermembran durch Anströmen mit einer Kontaktflüssigkeit verstanden. Polyvinypyrrolidon kann z.B. durch seine Hydrophilie durch wässrige Flüssigkeiten, wie z.B. auch Blut, aus dem Membranmaterial oder von der Oberfläche der Hohlfasermembran herausgelöst und ausgespült werden. Bei einer Elutionstestmethode werden Hohlfasermembranen auf der mit Polyvinylpyrrolidon beschichteten Oberfläche der Hohlfasermembran mit einem Extraktionsmittel, insbesondere Wasser, angeströmt. Der aus der Hohlfasermembran eluierte Teil von Polyinylpyrrolidon kann aus der Konzentration des Polyvinylpyrrolidons im Extrakt bestimmt werden.

Die Elution von Polyvinylpyrrolidon aus einer erfindungsgemäßen Hohlfasermembran ist damit geringer als die Elution, die an einer Hohlfasermembran gemessen wird, deren Membranmaterial aus Polysulfon und Polyvinylpyrrolidon besteht, aber kein zusätzliches fettlösliches Vitamin und keine zusätzliche Beschichtung mit Polyvinylpyrrolidon aufweist. Es ergibt sich dadurch der Vorteil, dass die Hydrophilie der Oberfläche der Hohlfasermembran, insbesondere der inneren Oberfläche der Hohlfasermembran, durch Beschichten mit Polyvinylpyrrolidon gesteigert werden kann, gleichzeitig aber die Elution gegenüber Vergleichshohlfasermembranen gesenkt werden kann. Insbesondere ergibt sich daraus aus medizinischer Sicht ein Vorteil, da während der extrakorporalen Blutbehandlung weniger Polyvinylpyrrolidon von der Hohlfasermembran eluiert wird und in den menschlichen Organismus eingetragen wird. Der Eintrag von Polyvinylpyrrolidon in den menschlichen Organismus ist insbesondere deshalb als kritisch anzusehen, da Polyvinylpyrrolidon ab einem gewissen Molekulargewicht nicht mehr vom menschlichen Körper metabolisiert wird und nur bedingt über die Nieren ausgeschieden wird.

In einer weiteren Ausführungsform der erfindungsgemäßen Hohlfasermembran beträgt der Gesamtgehalt an Polyvinylpyrrolidon, der in der Hohlfasermembran vorhanden ist, 3 bis 5 Gew.%, insbesondere mehr als 3 Gew.%, insbesondere mehr als 3,5 Gew.%, insbesondere weniger als 5 Gew.%, weiter insbesondere weniger als 4,5 Gew.%. Mit einer solchen Zusammensetzung ist die Einstellung eines ausgewogenen Eigenschaftsprofils aus mechanischer Stabilität, geeigneter Porosität und guter Hydrophilie möglich.

Die erfindungsgemäße Hohlfasermembran weist gemäß einer Ausführungsform an der Oberfläche des Lumens ein höheres gewichtsmittleres Molekulargewicht (Mw) des PVP auf als das des PVP im Volumen der Membran. Es hat sich gezeigt, dass ein niedriges Molekulargewicht des PVP im Volumen der Membran anzustreben ist, da solche Kompositionen einen hohen PVP-Anteil ermöglichen und somit im Volumen besonders hydrophil sind. Gleichzeitig zeigte sich, dass ein besonders hohes Molekulargewicht des PVP auf der Oberfläche des Lumens der Membran zu einer hohen Belegung und somit zu einer besonders optimierten Oberflächenhydrophilie führt. Solche Membranen sind im besonderen Maße biokompatibel und weisen eine geringe Elution von PVP nach Alterung auf.

Bei einer weiteren Ausführungsform der Hohlfasermembran weist die Membran ein gewichtsmittleres Molekulargewicht (Mw) des PVP der Oberfläche des Lumens auf, welches größer als 1.000.000 g/mol, z.B. 1.005.000 g/mol oder 1.100.000 g/mol, insbesondere größer als 2.000.000 g/mol, bevorzugt größer als 1.000.000 g/mol bis 3.000.000 g/mol, weiter bevorzugt größer als 2.000.000 g/mol bis 3.000.000 g/mol ist. Es hat sich gezeigt, dass ein gewichtsmittleres Molekulargewicht des PVP im Volumen der Membran von weniger als 1.000.000 g/mol, z. B. 995.000 g/mol oder 900.000 g/mol, bevorzugt 500.000 g/mol bis weniger als 1.000.000 g/mol, vorteilhaft ist. Bevorzugt beträgt das Verhältnis der gewichtsmittleren Molekulargewichte des PVP im Koagulationsmittel zu dem gewichtsmittleren Molekulargewicht des PVP in der Spinnmasse mindestens 1,2, bevorzugt mindestens 2, weiter bevorzugt 1,2 bis 3, weiter bevorzugt 2 bis 3, da solche Kompositionen einen hohen PVP-Anteil ermöglichen und somit im Volumen besonders hydrophil sind. Gleichzeitig zeigte sich, dass ein besonders hohes gewichtsmittleres Molekulargewicht des PVP auf der Oberfläche des Lumens der Membran zu einer hohen Belegung und somit zu einer besonders optimierten Oberflächenhydrophilie führt. Solche Membranen sind im besonderen Maße biokompatibel und weisen eine geringe Elution von PVP nach Alterung auf.

Eine weitere erfindungsgemäße Hohlfasermembran weist ein Verhältnis der Siebkoeffizienten für Albumin, gemessen nach 5 min, zu dem Siebkoeffizienten gemessen nach 30 min, gemäß der Messmethode **"Bestimmung des Plasma-Albumin-Siebkoeffizienten"** der Beschreibung, von weniger als 7, insbesondere von weniger als 5, auf.

Eine weitere erfindungsgemäße Hohlfasermembran weist ein Verhältnis der Siebkoeffizienten für Albumin, gemessen nach 5 min, zu dem Siebkoeffizienten gemessen nach 10 min, gemäß der Messmethode **"Bestimmung des Plasma-Albumin-Siebkoeffizienten"** der Beschreibung, von weniger als 3, insbesondere von weniger als 2, auf.

Eine Membran, die in der Dialyse Verwendung finden soll, wird durch Optimierung der Herstellparameter üblicherweise auf einen therapeutisch anzustrebenden Siebkoeffizienten eingestellt, der gemäß dem in der Beschreibung angegebenen Test nach 30 min gemessen wird. Nach diesem Zeitpunkt ist weitgehend ein Gleichgewicht des Siebkoeffizienten eingestellt, der dann für einen Dialysezeitraum von 4 Stunden oder mehr konstant bleibt. Zu Anfang des durchgeführten Tests und auch zu Anfang einer Dialyse wird die Membran jedoch mit Blutbestandteilen, insbesondere mit Proteinen, belegt, so dass der Siebkoeffizient anfangs abnimmt. Ist die Membranbelegung stärker, tritt ein ausgeprägterer Abfall des Siebkoeffizienten auf und die Membran muss "offener" gestaltet werden. Dies führt zu einem stärkeren Albuminverlust in den ersten Minuten der Dialyse. Erfindungsgemäße Membranen weisen einen geringeren Abfall des Siebkoeffizienten zu Beginn des Tests bzw. zu Beginn einer Dialyse auf und führen daher zu einem geringeren Albuminverlust, was therapeutisch erstrebenswert ist.

Eine weitere Hohlfasermembran weist ein Membranmaterial auf, das ein hydrophobes und ein hydrophiles Polymer umfasst, und ist gekennzeichnet durch einen Plättchenverlust, gemessen nach der Methode "Bestimmung des "Platelet Loss" (Absolutmethode)", von weniger als 50 %, bevorzugt weniger als 30 %, besonders bevorzugt weniger als 20 %. Plättchenverluste entstehen durch Adsorption an die Oberfläche des Lumens der Membran, was zu einer Beeinträchtigung des Blutes des Patienten führt und gleichzeitig zu einer Verringerung des zur Verfügung stehenden Querschnittes des Lumens, was ggf. zu erhöhtem Druckabfall auf der Blutseite während der Dialyse führen kann. Tritt hoher Plättchenverlust auf, so können einzelne Fasern komplett verstopfen und die Leistung des Filters herabsetzen. In der Therapie muss diesem Effekt durch eine Zugabe von Heparin entgegengewirkt werden, aufgrund des geringeren Plättchenverlustes ist bei erfindungsgemäßen Filtern eine geringere Heparinzugabe bei zumindest einigen Patienten möglich.

## Beschreibung der Erfindung anhand von Beispielen

Die Erfindung wird im Folgenden anhand von Messmethoden und Ausführungsbeispielen beschrieben, ohne darauf eingeschränkt zu sein.

## Messmethode zur Bestimmung der Elution von Polyvinylpyrrolidon

Hohlfasermembranfilter werden auf eluierbare Anteile von Polyvinylpyrrolidon untersucht. Dazu werden die Hohlfasermembranfilter mit einem Extraktionsmittel bei einer festgelegten Temperatur über einen festgelegten Zeitraum gespült. Der Extrakt wird anschließend auf den Gehalt von Polyvinylpyrrolidon untersucht. Dazu werden Hohlfasermembranfilter nach folgender Vorschrift aufgebaut:

Es wird ein Hohlfasermembranfilter (Dialysator) mit 10752 Hohlfasermembranen, die einen Innendurchmesser von 185 μm und eine Wandstärke von 35 μm aufweisen, verwendet. Der Innendurchmesser des Filtergehäuses beträgt 34 mm. Die für die Messung der Elution relevante Länge der Hohlfasermembranen beträgt 258 mm. Die Hohlfasermembranen sind an den Enden in dem Hohlfasermembranfilter so vergossen, dass ein erster Raum entsteht, der das Innere der Hohlfasermembranen umfasst ("Blutraum") und ein zweiter Raum ("Dialysatraum") entsteht, der den Raum zwischen den Hohlfasermembranen umfasst. Als Vergussmaterial wird Polyurethan der Fa. Elastogran (Polyol C6947 und Isocyanat 136 20) verwendet. Die Vergusshöhe an jedem Bündelende beträgt 22 mm. Als Extraktionsmittel dient Wasser. Es werden 1000 ml auf 37°C temperierten deionisierten Wassers durch den ersten Raum der Hohlfasermembranfilter, der das Innere der Hohlfasermembranen umfasst, über zwei Anschlüsse des Hohlfasermembranfilters gespült. Die weiteren zwei Anschlüsse des Hohlfasermembrafilters werden verschlossen. Der Spülvorgang wird im Rezirkulationsmodus vorgenommen. Dazu wird ein auf 37°C temperiertes Wasserbad bereitgestellt. Über eine Pumpe wird dem Hohlfasermembranfilter über einen ersten Anschluss temperiertes Wasser aus dem Wasserbad zugeführt. Es wird der erste Raum des Hohlfasermembranfilters durchspült, das Wasser über einen zweiten Anschluss aus dem Hohlfasermembranfilter abgeführt und in das Wasserbad zurückgeleitet. Das Durchspülen im Rezirkulationsmodus wird für 5 h mit einer Förderleistung von 200 ml/min durchgeführt.

Das nach dieser Methode eluierbare Polyvinylpyrrolidon konzentriert sich dabei in dem Wasserbad auf. Die Konzentration von Polyvinylpyrrolidon in dem Wasserbad kann photometrisch bestimmt werden. Zur photometrischen Bestimmung wird die orangebraune Farbreaktion des Polyvinylpyrrolidons mit Jod/Jodkalium in einer schwach sauren Lösung genutzt (spektralphotometrische Bestimmung nach Müller oder Breinlich).

Für die Durchführung werden 10 ml des Extrakts mit 5,0 ml Zitronensäure-Lösung und 2,0 ml $KI_3$-Lösung versetzt, gemischt und 10 min bei Raumtemperatur miteinander reagiert. Anschließend wird die Extinktion der Probelösung bei 470 nm bestimmt. Der Gehalt wird mit Hilfe einer zuvor ermittelten Kalibrierung aus der gemessenen Extinktion bestimmt. Für die Kalibrierung wird ein PVP des Typs K81-86 verwendet.

Zusätzlich wird die PVP-Elution nach einer beschleunigten Alterung bestimmt. Dazu wird jeweils ein Dialysator bei 80°C und einer relativen Luftfeuchte von < 5 % für eine Zeit von 30, 60 und 120 Tagen in einem Trockenschrank gelagert. Nach dieser Lagerung wird die PVP-Elution bestimmt. Die extrahierte Menge an PVP wird auf die Einzelfaser bezogen, als Wert wird jeweils die Menge in $10^{-7}$ mg pro Einzelfaser, bestimmt nach der obigen Methode, angegeben.

**Messmethode zur Bestimmung des "Platelet Loss" und der Komplementaktivierung (Vergleichsmethode)**

Für die Bestimmung des Plättchenverlustes ("patelet loss") und der Komplementaktivierung wird gesunden Spendern ohne Medikamenteneinnahme, die die Koagulation des Blutes oder die Plättcheneigenschaften beeinflussen könnte, 450 ml humanes Vollblut mit einer 17G (1,5 mm) Nadel entnommen. Im Blutbeutel wird 750 IU Heparin, verdünnt in 50 ml physiologischer Kochsalzlösung, vorgelegt, so dass eine Heparinkonzentration von 1,5 IU pro ml der Mischung aus Blut und Salzlösung entsteht. Innerhalb von 30 min nach der Blutspende wird die Methode zur Bestimmung des Plättchenverlusts gestartet.

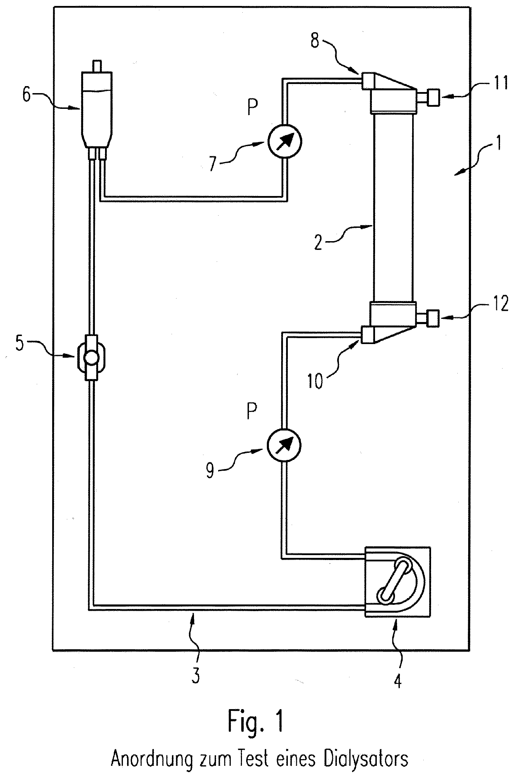

Für die zu testenden Hohlfasermembranen wird nach der schematischen Darstellung gemäß der Figur 1 eine Apparatur (1) aufgebaut, um den Plättchenverlust zu bestimmen. Die Apparatur umfasst einen Dialysator (2), hergestellt wie zuvor beschrieben, mit den darin aufgenommenen, zu untersuchenden Hohlfasermembranen. Weiterhin umfasst die Apparatur ein Schlauchsystem (3), eine Schlauchpumpe (4), eine Stelle zur Entnahme von Blutproben (5), ein Reservoir für Blut (6), einen Drucksensor (7) am Bluteinlass (8) des Dialysators (2) und einen Drucksensor (9) am Blutauslass (10) des Dialysators (2). Für die Durchführung der Bestimmung wurden 200 ml des, wie vorab beschriebenen, heparinisierten Blutes verwendet. Das Blut wurde durch das Schlauchsystem (3) (Material: PVC, Hersteller Fresenius Medical Care, Deutschland) durch den Dialysator (2) mit Hilfe der Schlauchpumpe (4) (Hersteller: Fresenius Medical Care, Deutschland) durch die Apparatur (1) gefördert. Für jede Messung wurde ein neues Schlauchsystem verwendet. Die gesamte Apparatur (1) wurde vor der Messung für 30 min mit einer 0,9 %igen (w/v) Salzlösung gespült. Zur Befüllung der Apparatur mit Blut wird die Spüllösung bei geringer Pumpendrehzahl mit Blut, das in die Apparatur eingeleitet wird, verdrängt und abgeleitet, bis die Apparatur mit reinem Blut befüllt war. Die Füllmenge mit Blut betrug 200 ml. Die verdrängte Lösung wurde verworfen.

Zur Vermeidung einer Ultrafiltration während des Versuchs wurde die Dialysatseite vorab mit einer 0,9 %igen Salzlösung über Zugänge (11, 12) am Dialysator gefüllt und danach verschlossen. Die Durchführung des Blättchenverlustes erfolgt bei 37°C, z.B. in einem Brutschrank (Fa. Memmert, Deutschland), über einen Zeitraum von 180 min, wobei zu Messbeginn, nach 30, 60, 120 und 180 min Proben an der Stelle zur Entnahme von Blutproben (5) entnommen werden. Der Druck am Bluteinlass (8) und am Blutauslass (9) wird gemessen, um einen gleichbleibenden Verlauf der Bedingungen während der Durchführung der Bestimmung sicherzustellen. Beim Auftreten von signifikanten Druckänderungen muss die Messung verworfen werden. Das Blut wurde mit einer Volumenrate von 200 ml/min durch die Apparatur gepumpt.

Die Hemokompatibilität wurde anhand der Parameter Komplementaktivierung (C5a) und anhand des Plättchenverlustes bestimmt. Der Plättchenverlust wurde in einer Dreifachbestimmung mit einem automatischen Hematologieanalysator (K4500 Sysmex, Norderstedt, Deutschland) bestimmt.

Die Komplementaktivierung wurde mit einer Doppelbestimmung mit Hilfe eines ELISA-Test-Kits (EIA-3327) der Fa. DRG Instruments, Marburg, Deutschland bestimmt. Als Messparameter diente der Faktor C5a, der durch proteolytische Aktivierung des Faktors C5 entsteht. Neben dem Faktor C5a entsteht dabei ein weiteres Fragment, das als Faktor C5b bezeichnet wird.

Die Auswertung der Parameter Komplementaktivierung und Plättchenverlust wird so durchgeführt wie in der Publikation "Score Model for the Evaluation of Dialysis Membrane Hemocompatibility", Erlenkötter et al., Artificial Organs 32(12):962-998, 2008 gemäß der Formeln (1, Komplementaktivierung) und (2, Plättchenverlust) beschrieben. Für die Bestimmung des Plättchenverlustes beträgt der Messzeitraum jeweils die ersten 60 min des Gesamtversuches. Deshalb gelten für Formel 2 folgende Bedingungen:

$$I_{PLT} = \frac{\int_{t1}^{t2} \Delta c_{PLT}(t)dt}{T_{II}} \quad \text{Formel (2)}$$

mit $I_{PLT}$ = mittlerer Plättchenverlust ( %), t1= o min, t2 = 60 min, $\Delta$ c $_{PLT}$ = Plättchenkonzentration, $T_{II}$ = 60 min.

Bei den Messungen wurde jeweils mit der zweiten Hälfte der Blutspende ein weiterer Filter (FX60 der Fa. Fresenius Medical Care, Deutschland) als Referenz mitgemessen und die Messergebnisse relativ (in %) zu diesem Vergleichsfilter bestimmt. Somit konnten die natürlichen starken Schwankungen der Blutreaktion verschiedener Spender rechnerisch ausgeglichen werden. Beispiele und Vergleichsbeispiele wurden mit denselben Chargen der Rohmaterialien hergestellt.

**Messmethode zur Bestimmung des "Platelet Loss" (Absolutmethode)**

Die Messung wird analog der Vergleichmethode zur Bestimmung des "Platelet Loss" durchgeführt, die erhaltenen Messergenisse werden jedoch absolut verwendet und nicht in einen Vergleich zum Filter FX60 gesetzt. Zusätzlich wird ein anderer Testfilteraufbau verwendet: Die verwendete Membran hat einen Innendurchmesser von 210 μm und eine Wandstärke von 40 μm, sie wird zu einem Bündel von 10752 Fasern zusammengeführt und in ein Filtergehäuse mit einem Innendurchmesser von 38,4 mm gebracht. Der Filter wird in gleicher Weise vergossen wie vorher beschrieben, so dass die gleiche effektive Länge der Faser zur Verfügung steht. Danach wird der jeweilige Filter in gleicher Weise wie zuvor beschrieben beblutet und der Parameter Plättchenverlust bestimmt, jedoch nicht ins Verhältnis zu einem Referenzfilter gesetzt.

**Messmethode zur Bestimmung des Zeta-Potentials**

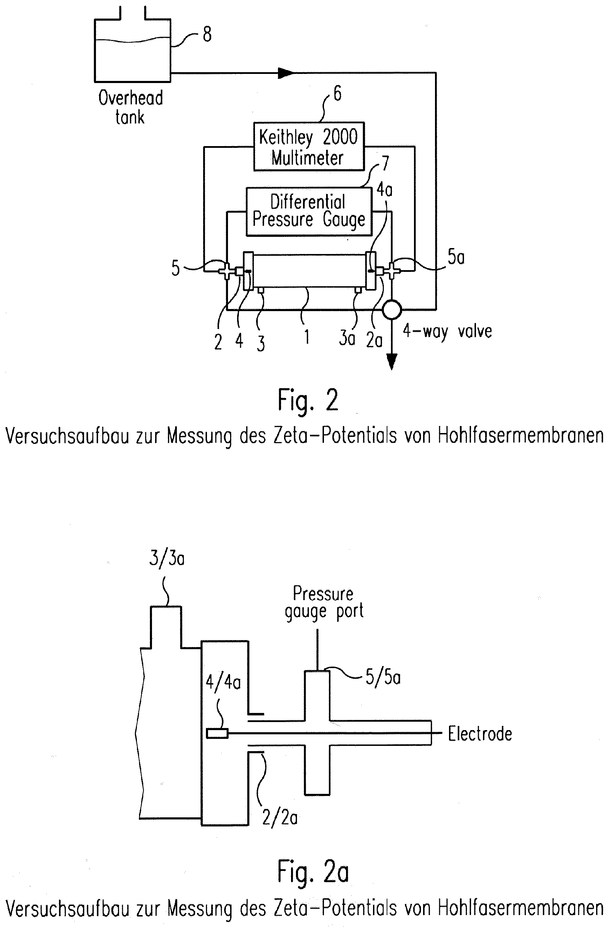

Für die Bestimmung des Zeta-Potentials der untersuchten Hohlfasermembranen wird ein Hohlfasermembranfilter (Dialysator) mit 10752 Hohlfasermembranen, die einen Innendurchmesser von 185 μm und eine Wandstärke von 35 μm aufweisen, verwendet. Der Innendurchmesser des Filtergehäuses beträgt 34 mm. Die für die Messung des Zeta-Potentials relevante Länge der Hohlfasermembranen beträgt 258 mm. Die Hohlfasermembranen sind an den Enden in dem Hohlfasermembranfilter so vergossen, dass ein erster Raum entsteht, der das Innere der Hohlfasermembranen umfasst und ein zweiter Raum entsteht, der den Raum zwischen den Hohlfasermembranen umfasst. Als Vergussmaterial wird Polyurethan der Fa. Elastogran (Polyol C6947) und Isocyanat 136 20) verwendet. Die Vergusshöhe an jedem Bündelende beträgt 22 mm. Es wird eine Apparatur gemäß der Fig. 2/2a zur Messung verwendet. Der Hohlfasermembranfilter (1) weist Fluidzugänge (2, 2a, 3, 3a) zu dem jeweils ersten und zweiten Raum des Hohlfasermembranfilters (1) auf. Die Fluidzugänge zum ersten Raum des Hohlfasermembranfilters (1) werden mit je einer Ag/AgCl- Elektrode (4, 4a) und einem Zugang für die Druckmessung (5, 5a) gemäß der Fig. 2a versehen. Die Fluidzugänge (3, 3a) zum zweiten Raum des Hohlfasermembranfilters (1) werden dicht verschlossen, so dass der zweite Raum des Hohlfasermembranfilters unbefüllt bleibt. Zwischen beiden Elektroden wird somit die Spannungsdifferenz $\Delta E_z$ (mV) mit Hilfe eines Spannungsmessgerätes (6) aufgezeichnet. Zwischen den Zugängen für die Druckmessung (5, 5a) wird der Druckabfall $\Delta P$ ($N/m^2$) mit Hilfe eines Druckmessgerätes (7) aufgezeichnet. Die Prüfflüssigkeit besteht aus einer 1 mmolaren Lösung von KCl in Wasser mit einem pH-Wert von 7.4 und wird in einem Reservoir (8) vorgelegt, das ca. 1000 mm oberhalb des Filters platziert wird. Die pH- Wert-Einstellung erfolgt nach folgender Vorschrift: zu 100 Litern der KCl- Lösung werden 50 mg $K_2CO_3$ gegeben. Bei geöffnetem Behälter wird solange gerührt, bis sich ein pH-Wert von 7,4 einstellt. Dann wird der Behälter dicht verschlossen. Die Messung wird bei einer Temperatur von 23°C +/- 2°C durchgeführt.

Für die Messung des Zeta-Potentials wird die Prüfflüssigkeit durch einen ersten Fluidzugang (2) in den ersten Raum des Hohlfasermembranfilters eingeströmt, der den inneren Raum der Hohlfasermembranen umfasst und wird durch einen zweiten Fluidzugang (2a) am Hohlfasermembranfilter, der mit dem inneren Raum der Hohlfasermembranen in Verbindung steht, wieder aus dem Dialysator ausgeleitet. Der Hohlfasermembranfilter wird in dieser Anordnung zunächst 10 min mit der Prüfflüssigkeit gespült, bis sich ein stabiler Wert eingestellt hat, ggf. wird weitere 5 min, gespült. Die Druckdifferenz und die Spannungsdifferenz wird zeitgleich an der Druckmessvorrichtung bzw. dem Multimeter abgelesen und daraus das Zeta-Potential berechnet. Zur Erhöhung der Messgenauigkeit ist vorgesehen, dass nach der Messwertaufnahme die beiden 4-Wege-Ventile so umgeschaltet werden, dass sich ein umgekehrter Fluss der Prüfflüssigkeit durch den inneren Raum der Hohlfasermembranen ergibt. Der Messwert für das Zeta-Potential wird dann aus dem Mittelwert der Messungen in beiden Flussrichtungen gebildet. Die Berechnung des Zeta-Potentials erfolgt nach folgender Gleichung:

$$\zeta = \frac{\eta \cdot \Lambda o \cdot d\, Ez}{\varepsilon o \cdot \varepsilon r \cdot d\, \Delta P}$$

mit

$\zeta$ = Zeta-Potential (mV)
$\eta$ = Lösungsviskosität (0,001 $Ns/m^2$)
$\Lambda_o$ = Leitfähigkeit der Lösung (A/(V*m))
$\varepsilon_o$ = Permittivität des Vakuums (8,85 * $10^{-12}$ A * s /(V * m)
$\varepsilon_r$ = relative Permittivität der Lösung ( 80 )
$E_Z$= Strömungspotential ( mV)
$\Delta_P$ = Druckdifferenz ( N /$m^2$ ).

**Messmethode zur Bestimmung des Kontaktwinkels** $\theta$

Der Kontaktwinkel einer Hohlfasermembran wurde nach der Kapillarmethode bestimmt, wobei die Hohlfasermembran als Kapillare dient. Die Hohlfasermembran wurde in einen Messstand eingespannt. In die am Boden des Messstandes angeordnete Wanne wurde deionisiertes Wasser, angefärbt mit 0,25 mg/ml Methylenblau, eingefüllt. Die Hohlfasermembran, die zuvor mit einem Rasiermesser mit einer neuen Schnittkante quer zur Längsausdehnung versehen wurde, wurde in die Lösung eingetaucht und die Kapillarhöhe h wurde nach einer Wartezeit von 20 min bestimmt, indem die Höhe der eingefärbten Lösung in der Hohlfasermembran oberhalb des Flüssigkeitsstandes der Testflüssigkeit in der Wanne bestimmt wurde. Nach jeder Messung wurde eine neue Hohlfasermembran verwendet. Der Innenradius r jeder Hohlfasermembran wurde lichtmikroskopisch an der Schnittkante ermittelt.

Die Young-Laplace-Gleichung für den Kapillardruck kann für die Berechnung des Kontaktwinkels verwendet werden:

$$\rho g h = (2\gamma\cos\theta)/r$$

Die Gleichung kann zur Bestimmung des Kontaktwinkels bei gegebenem Innenradius, der Kapillarhöhe und den bekannten Konstanten wie folgt bestimmt werden:

$$\mathrm{Arcos}(\rho g h r/2\gamma) = \theta$$

wobei

$\rho$ = Dichte von Wasser bei 25°C: 0,997 $kg/m^3$
g = Erdbeschleunigung 9,8 $m/s^2$
h = Kapillarhöhe, m
$\gamma$ = Oberflächenspannung von Wasser bei Raumtemperatur, 0,0728 N/m
r = Kapillarradius
$\theta$ = der zu bestimmende Kontaktwinkel

Der Kontaktwinkel wurde aus dem Mittelwert von 12 Messungen bestimmt.

**Messmethode zur Bestimmung des Polyvinylpyrrolidongehalts in der Hohlfasermembran**

Die Bestimmung des PVP-Gehaltes der Hohlfasermembran wird mit Hilfe der IR-Spektroskopie durchgeführt. Dazu wird eine Probe aus Hohlfasermembranen zunächst 2 Stunden im Trockenschrank bei 105°C getrocknet. Dann wird 1 g der Hohlfasermembranen in Dichlormethan aufgelöst. Zusätzlich werden Kalibierstandards unter Verwendung von getrocknetem PVP, welches ebenfalls in Dichlormethan aufgelöst wird, erstellt. Dabei wird ein Konzentrationsbereich von ca. 1 % bis 10 % PVP in der Hohlfaser abgedeckt. Die Lösungen werden jeweils in eine Flüssigküvette mit einer Schichtdicke von 0,2 mm gefüllt. Zur Auswertung wird die Absorptionsbande der C-O-Carbonylschwingung herangezogen.

**Messmethode zur Bestimmung des Polyvinylpyrrolidon in einer oberflächennahen Schicht (XPS)**

Der Gehalt des Polyvinylpyrrolidons in der oberflächennahen Schicht wurde mit Hilfe der Photoelektronenspektroskopie (XPS oder ESCA) bestimmt. Mit Hilfe dieser Methode kann der Anteil des Polyvinylpyrrolidons in einer Schicht von ca. 5 - 10 nm bestimmt werden. Diese Schicht, die mit Hilfe der XPS- Methode beprobt wird, wird im Folgenden "*oberflächennahe Schicht*" genannt. Sie wird durch die Messbedingungen festgelegt.

Eine Hohlfasermembran wird mit Hilfe eines Skalpells oder sonstigen scharfen Messers aufgetrennt, so dass die innere Oberfläche und damit die selektive Schicht der Hohlfasermembran freiliegt. Diese Probe wird auf einem Probenteller fixiert und in den Probenraum verbracht. Die Messbedingungen werden wie folgt festgelegt:

- Apparat: Thermo VG Scientific, Typ K-Alpha
- Anregungsstrahlung: monochromatische Röntgenstrahlung, Al K$\alpha$, 75 W
- Durchmesser des Probenflecks: 200$\mu$m
- Passenergie: 30 eV
- Winkel zwischen Quelle und Analysator: 54°
- Spektrale Auflösung für ein Ag3d- Signal: 0,48 eV:
- Angelegtes Vakuum:$10^{-8}$ mbar
- Die Aufladung wurde mit Hilfe einer Flood-Gun kompensiert.

Der Gehalt an PVP in der oberflächennahen Schicht wurde mit Hilfe der gefundenen Werte in Atom % von Stickstoff (N) und Schwefel (S) durch folgende Gleichung ermittelt:

$$\text{Gehalt des PVP [in Massen \%]} = 100 * (N*111)/(N*111+ S*442)$$

Diese Gleichung ist gültig für die Verwendung von Polysulfon auf Bisphenol-A-Basis, für Polyethersulfon ist folgende Gleichung anzuwenden:

$$\text{Gehalt des PVP [in Massen \%]} = 100 * (N*111)/(N*111+ S*232)$$

Für andere Polysulfone muss das dem Schwefel zuzuordnende Molekulargewicht der Monomereinheit ermittelt werden, bei Copolymeren muss der Anteil des schwefelhaltigen Monomers am Copolymer berücksichtigt werden.

Es wird jeweils eine Bestimmung an 3 Hohlfasermembranen durchgeführt und der Mittelwert dieser Messungen berechnet.

**Messmethode zur Bestimmung des Peakhöhenverhältnisses von $CNO^-$ und $SO_2^-$ mittels TOF-SIMS in einer Oberflächenschicht**

Die Zusammensetzung der Oberflächenschicht wurde mit Hilfe der Sekundärionenmassenspektroskopie bestimmt. Als Ionendetektor wurde ein Flugzeitmassenspektrometer (Time-of-flight, TOF) verwendet. Die Probe wurde in gleicher Weise wie bei der Bestimmung der oberflächennahen Schicht präpariert und in die Probenkammer eingeschleust. Für die Messungen wurde das Modell TOF-SIMS IV der Fa. ION-TOF (Münster, Deutschland) verwendet. Die Messungen wurden bei der Fa. nanoAnalytices (Münster, Deutschland) durchgeführt. Die Messmethode ermittelt die relative chemische Zusammensetzung der Oberflächenschicht einer Probe, repräsentiert durch die erste Monolage bzw. die ersten 1 - 3 Monolagen der Oberfläche. Die wesentlichen Messparameter waren wie folgt:

- Massenauflösung: m/dm > 8000
- Massenbereich < 3000 m/z
- Abstand zwischen Probe und Quelle: 2 mm
- Primärionen: $Bi^+$, Beschleunigungsspannung 30 kV
- Nachbeschleunigung: 30 kV
- Sekundärionenpolarität: negativ und positiv
- Primärionendosis: 2,65 * $10^8$ Ionen pro Messung

- Größe der beprobten Fläche: 10.000 $\mu m^2$ (100 × 100 $\mu$m)
- Angelegtes Vakuum:10-8 mbar
- Pulsweite: 10 ns (ungebunched)
- 0,5 ns (gebunched)
- Bunching: Ja (hochauflösende Messung)
- Ladungsneutralisierung: Ja

Bei der Wahl der Messparameter wird darauf geachtet, dass die Peakhöhe des Ions $CNO^-$ zwischen 0,1 und 2 * $10^5$ counts per channel eingestellt ist.

Zur Auswertung wurde das Spektrum der Anionen herangezogen, wobei bei den jeweiligen Proben die Ionen $CNO^-$ mit der Masse 42 und $SO_2^-$ mit der Masse 64 ausgewertet wurden. $CNO^-$ repräsentiert dabei ein Signal des PVP, $SO_2^-$ repräsentiert ein Signal des Polysulfons. Das Anionenspektrum wird aufgezeichnet und die jeweiligen Peakhöhen H, die die entsprechenden Massen repräsentieren, gemessen. Diese Peakhöhen H werden dann ins Verhältnis zueinander gesetzt, der ermittelte Wert stellt einen Messparameter für das Verhältnis des PVP zum Polysulfon dar.

$$\text{Peakhöhenverhältnis in der Oberflächenschicht} = \frac{\text{Peakhöhe H (CNO)}}{\text{Peakhöhe H (SO2)}}$$

Es werden jeweils 3 Membranen gemessen und der Mittelwert berechnet.

**Messmethode zur Bestimmung des Plasma-Albumin- Siebkoeffizienten**

Die Messung des Albumin-Siebkoeffizienten einer Hohlfasermembran wird in Anlehnung an die DIN EN ISO 8637:2014 an einem fertig aufgebauten Hohlfasermembranfilter durchgeführt. Dazu wird ein Filter mit 10752 Hohlfasermembranen mit einem Innendurchmesser von 185 $\mu$m und einer Wandstärke von 35 $\mu$m verwendet. Die aktive Länge der Hohlfasermembran beträgt 235 mm. Unter einer aktiven Länge einer Hohlfasermembran ist die Länge der Hohlfasermembran ohne Verguss zu verstehen, die für die Bestimmung der Permeationseigenschaften, wie Siebkoeffizient, Clearance und Ultrafiltrationskoeffizient, zur Verfügung steht. Der Innendurchmesser des Hohlfasermembranfilters beträgt in der Mitte 34 mm. Der Hohlfasermembranfilter weist ansonsten den gleichen Aufbau auf, wie er in "Messmethode Zeta-Potential" beschrieben ist. Zur Messung wird ein Humanplasma in Anlehnung an die Norm DIN EN ISO 8637:2014 zur Bestimmung des Siebkoeffizienten verwendet. Es wird also der "Plasma-Siebkoeffizient" des Albumins bestimmt. Die Plasmalösung wird durch die Fluidzugänge, durch den ersten Raum des Hohlfasermembranfilters, der das Innere der Hohlfasermembranen umfasst, mit einem Fluss von 500 ml/min durchgeleitet. Im zweiten Raum des Hohlfasermembranfilters wird über die Fluidzugänge ein Fluss von 100 ml/min von reinem Wasser im Gegenstrom eingestellt. Nach 5, 10 und 30 min wird die Konzentration des Albumins am ersten und zweiten Fluidzugang des ersten Raums des Hohlfasermembranfilters und auf der Filtratseite ermittelt und daraus der Siebkoeffizient gemäß der Norm bestimmt. Als Analysengerät findet das Modell Cobas Integra 400 plus der Fa. Roche Diagnostics GmbH, Mannheim Verwendung. Die Durchführung der Messung erfolgt mit Hilfe des Testes ALBT2 in der Applikation für Urin.

**Beispiel 1: Herstellung einer erfindungsgemäßen Hohlfasermembran**

Eine Spinnlösung bestehend aus 16 Gewichtsteilen Polysulfon (P3500 der Fa. Solvay), 4,3 Gewichtsteilen Polyvinylpyrrolidon (K81/ 86 der Firma Ashland) und 79,7 Gewichtsteilen DMAc wird unter Rühren, Erwärmen auf 60°C und Entgasen zu einer homogenen Spinnmasse verarbeitet. Anschließend wird der Spinnmasse $\alpha$-Tocopherol (Fa. Sigma Aldrich) zugegeben, so dass der Anteil an $\alpha$-Tocopherol an der Gesamtmasse der Spinnmasse 0,01 Gew.% beträgt. Für die Herstellung des Koagulationsmittels werden 35 Gew.% DMAc und 65 Gew.% Wasser gemischt und Polyvinylpyrrolidon (K81/ 86 der Firma Ashland) zugegeben, so dass der Anteil von Polyvinylpyrrolidon 1 g pro kg (1000 ppm) Koagulationsmittel beträgt. Die Spinnmasse wird durch eine Ringspaltdüse mit dem zentral geführten Koagulationsmittel zu einem Spinnfaden mit einem Lumendurchmesser von 185 $\mu$m und einer Wandstärke von 35 $\mu$m verarbeitet. Das Koagulationsmittel wird im Inneren des hohlen Spinnfadens geführt. Die Temperatur der Ringspaltdüse beträgt 70°C. Der Spinnfaden wird durch einen Fällraum geführt, dessen Atmosphäre eine relative Feuchtigkeit von 100 % aufweist. Die Höhe des Fällspaltes beträgt 200 mm, es wird eine Durchlaufzeit durch den Fällspalt von 0,4 sec eingestellt. Der Spinnfaden wird dann in ein Fällbad bestehend aus Wasser eingeleitet, das auf 80°C temperiert ist und zu einer Hohlfasermembran ausgefällt. Anschließend wird die Hohlfasermembran durch Spülbäder, die auf eine Temperatur von 75°C bis 90°C temperiert sind, geleitet. Anschließend durchläuft die Hohlfasermembran einen Trocknungsprozess zwischen 100°C und 150°C. Die erhaltene Hohlfasermembran wird anschließend von einer Haspel aufgenommen und zu einer Fadenschar zusammengeführt. Aus der gehaspelten Fadenschar werden Hohlfasermembranbündel hergestellt.

Das Hohlfasermembranbündel wird nach bekannten Techniken zu Hohlfasermembranfiltern weiterverarbeitet. Der erhaltene Hohlfasermembranfilter wird nach einem Dampfsterilisationsverfahren, wie es in der Patentanmeldung DE 102016224627.5 beschrieben wird, sterilisiert. An den so sterilisierten Hohlfasermembranfiltern werden die Messmethoden zur Bestimmung des Zeta-Potentials, der Komplementaktivierung und des Platelet Loss durchgeführt. Die Ergebnisse der einzelnen Untersuchungen an Hohlfasermembranen, die nach dem Beispiel 1 gefertigt wurden, sind in Tabelle 1 dargestellt.

**Beispiel 2: Herstellung einer erfindungsgemäßen Hohlfasermembran**

Es wurden für die Herstellung einer erfindungsgemäßen Hohlfasermembran und der Filter die gleichen Ausgangsbedingungen wie in Beispiel 1 gewählt, mit dem Unterschied, dass der Anteil von Polyvinylpyrrolidon in dem Koagulationsmittel 1,5 g pro kg Koagulationsmittel (1500 ppm) betrug. An den so sterilisierten Hohlfasermembranfiltern werden die Messmethoden zur Bestimmung des Zeta-Potentials, der Komplementaktivierung und des Platelet Loss durchgeführt. Darüber hinaus werden sterilisierte Hohlfasermembranfilter einer beschleunigten Alterung bei 80°C für 30 bzw. 60 Tage unterzogen, wie in der "Messmethode zur Bestimmung der Elution von Polyvinylpyrrolidon" beschrieben. Nach der jeweiligen Alterung wurde die PVP-Elution bestimmt. Zur Messung des Kontaktwinkels, des Gehalts von PVP in der Hohlfasermembran, des Gehalts von Polyvinylpyrrolidon in der oberflächennahen Schicht und des Peakhöhenverhältnisses von $CNO^-$ und $SO_2^-$ mittels TOF-SIMS in der Oberflächenschicht werden entsprechend hergestellte Hohlfasermembranfilter geöffnet und Hohlfasermembranen zur Ermittlung der jeweiligen Werte entnommen. Die Ergebnisse der einzelnen Untersuchungen an Hohlfasermembranen, die nach dem Beispiel 2 gefertigt wurden, sind in Tabelle 1 dargestellt. Zusätzlich wurden die Albumin-Siebkoeffizienten nach 5 min,., 10 min, und 30 min bestimmt. Die Daten sind in Tabelle 2 angegeben. Weiter wurde nach obiger Vorschrift eine Membran hergestellt, jedoch mit den Dimensionen Lumendurchmesser 210 $\mu$m und Wandstärke 40 $\mu$m, welche zur Bestimmung des Plättchenverlustes nach der Absolutmethode verwendet wurde.

**Beispiel 3: Herstellung einer erfindungsgemäßen Hohlfasermembran**

Es wurden für die Herstellung einer erfindungsgemäßen Hohlfasermembran und der entsprechenden Filter die gleichen Ausgangsbedingungen wie in Beispiel 1 gewählt, mit dem Unterschied, dass der Anteil von Polyvinylpyrrolidon in dem Koagulationsmittel 2500 ppm Gewichtsteile beträgt. An den so sterilisierten Hohlfasermembranfiltern werden die Messmethoden zur Bestimmung des Zeta-Potentials, der Komplementaktivierung und des Platelet Loss durchgeführt. Die Ergebnisse der einzelnen Untersuchungen an Hohlfasermembranen, die nach dem Beispiel 3 gefertigt wurden, sind in Tabelle 1 dargestellt.

**Beispiel 4: Herstellung einer erfindungsgemäßen Hohlfasermembran**

Es wurden für die Herstellung einer erfindungsgemäßen Hohlfasermembran und der entsprechenden Filter die gleichen Ausgangsbedingungen wie in Beispiel 1 gewählt, mit dem Unterschied, dass der Anteil von Polyvinylpyrrolidon in dem Koagulationsmittel 3000 ppm Gewichtsteile betrug und die Konzentration des $\alpha$-Tocopherols (Vit.E) in der Spinnmasse 0,05 % (w/w) betrug. An den so sterilisierten Hohlfasermembranfiltern werden die Messmethoden zur Bestimmung des Zeta-Potentials, der Komplementaktivierung und des Platelet Loss durchgeführt. Darüber hinaus werden sterilisierte Hohlfasermembranfilter einer beschleunigten Alterung bei 80°C für 30 bzw. 60 Tage unterzogen, wie in der "Messmethode zur Bestimmung der Elution von Polyvinylpyrrolidon" beschrieben. Nach der jeweiligen Alterung wurde die PVP-Elution bestimmt. Die Ergebnisse der einzelnen Untersuchungen an Hohlfasermembranen, die nach dem Beispiel 4 gefertigt wurden, sind in Tabelle 1 dargestellt.

**Beispiel 5: Herstellung einer erfindungsgemäßen Hohlfasermembran**

Es wurden für die Herstellung einer erfindungsgemäßen Hohlfasermembran und der Filter die gleichen Ausgangsbedingungen wie in Beispiel 1 gewählt, mit dem Unterschied, dass der Anteil von Polyvinylpyrrolidon in dem Koagulationsmittel 1,5 g pro kg Koagulationsmittel (1500 ppm) betrug, wobei ein PVP des Tys K90 im Koagulationsmittel verwendet wurde. In der Spinnmasse wurde weiterhin ein PVP des Typs K81/86 verwendet. An den so sterilisierten Hohlfasermembranfiltern werden die Messmethoden zur Bestimmung des Zeta-Potentials durchgeführt. Zur Messung des Kontaktwinkels, des Gehalts von Polyvinylpyrrolidon in der oberflächennahen Schicht und des Peakhöhenverhältnisses von $CNO^-$ und $SO_2^-$ mittels TOF-SIMS in der Oberflächenschicht werden entsprechend hergestellte Hohlfasermembranfilter geöffnet und Hohlfasermembranen zur Ermittlung der jeweiligen Werte entnommen. Die Ergebnisse der einzelnen Untersuchungen an Hohlfasermembranen, die nach dem Beispiel 5 gefertigt wurden, sind in Tabelle 1 dargestellt. Membranen gemäß diesem Beispiel weisen einen besonders hohen Anteil von PVP an der Oberfläche des inneren Lumens auf, insbesondere verglichen mit dem Ausführungsbeispiel 2. Zusätzlich wurden die Albumin-Siebkoeffizienten nach 5 min, 10 min, und 30 min bestimmt. Die Daten sind in Tabelle 2 angegeben.

**Beispiel 6: Herstellung einer erfindungsgemäßen Hohlfasermembran**

Es wurden für die Herstellung einer erfindungsgemäßen Hohlfasermembran und der Filter die gleichen Ausgangsbedingungen wie in Beispiel 1 gewählt, mit dem Unterschied, dass der Anteil von Polyvinylpyrrolidon in dem Koagulationsmittel 2000 ppm betrug, wobei ein PVP des Tys K90 im Koagulationsmittel verwendet wurde. In der Spinnmasse wurde weiterhin ein PVP des Typs K81/86 verwendet. An den so sterilisierten Hohlfasermembranfiltern werden die Messmethoden zur Bestimmung des Zeta-Potentials durchgeführt. Zur Messung des Kontaktwinkels, des Gehalts von Polyvinylpyrrolidon in der oberflächennahen Schicht, und das Peakhöhenverhältnis von $CNO^-$ und $SO_2^-$ mittels TOF-SIMS in der Oberflächenschicht werden entsprechend hergestellte Hohlfasermembranfilter geöffnet und Hohlfasermembranen zur Ermittlung der jeweiligen Werte entnommen. Die Ergebnisse der einzelnen Untersuchungen an Hohlfasermembranen, die nach dem Beispiel 6 gefertigt wurden, sind in Tabelle 1 dargestellt. Membranen gemäß diesem Beispeil weisen einen bsonders hohen Anteil von PVP an der Oberfläche des inneren Lumens auf.

**Beispiel 7: Herstellung einer erfindungsgemäßen Hohlfasermembran**

Es wurden für die Herstellung einer erfindungsgemäßen Hohlfasermembran und der Filter die gleichen Ausgangsbedingungen wie in Beispiel 1 gewählt, mit dem Unterschied, dass der Anteil von Polyvinylpyrrolidon in dem Koagulationsmittel 1000 ppm betrug, wobei ein PVP des Typs K90 im Koagulationsmittel verwendet wurde. In der Spinnmasse wurde weiterhin ein PVP des Typs K81/86 verwendet. An den so sterilisierten Hohlfasermembranfiltern werden die Messmethoden zur Bestimmung des Zeta-Potentials durchgeführt. Zur Messung des Kontaktwinkels, des Gehalts von Polyvinylpyrrolidon in der oberflächennahen Schicht und des Peakhöhenverhältnisses von $CNO^-$ und $SO_2^-$ mittels TOF-SIMS in der Oberflächenschicht werden entsprechend hergestellte Hohlfasermembranfilter geöffnet und Hohlfasermembranen zur Ermittlung der jeweiligen Werte entnommen. Die Ergebnisse der einzelnen Untersuchungen an Hohlfasermembranen, die nach dem Beispiel 7 gefertigt wurden, sind in Tabelle 1 dargestellt.

**Vergleichsbeispiel 1: Herstellung einer Vergleichshohlfasermembran**

Es wurden für die Herstellung einer Vergleichs-Hohlfasermembran und der entsprechenden Filter die gleichen Ausgangsbedingungen wie in Beispiel 1 gewählt, mit dem Unterschied, dass der Anteil von Polyvinylpyrrolidon in dem Koagulationsmittel 1,5 g/kg Koagulationsmittel (1500 ppm) Gewichtsteile betrug und der Anteil des $\alpha$-Tocopherols in der Spinnmasse 0,00 % betrug. An den so sterilisierten Hohlfasermembranfiltern werden die Messmethoden zur Bestimmung der Komplementaktivierung und des Platelet Loss durchgeführt. Darüber hinaus werden sterilisierte Hohlfasermembranfilter einer beschleunigten Alterung bei 80°C für 30 bzw. 60 Tage unterzogen, wie in der "Messmethode zur Bestimmung der Elution von Polyvinylpyrrolidon" beschrieben. Nach der jeweiligen Alterung wurde die PVP-Elution bestimmt. Die Ergebnisse der einzelnen Untersuchungen an Hohlfasermembranen, die nach dem Vergleichsbeispiel 1 gefertigt wurden, sind in Tabelle 1 dargestellt.

**Vergleichsbeispiel 2: Herstellung einer Vergleichs-Hohlfasermembran**

Es wurden für die Herstellung einer Vergleichs-Hohlfasermembran und der entsprechenden Filter die gleichen Ausgangsbedingungen wie in Beispiel 1 gewählt, mit dem Unterschied, dass der Anteil von Polyvinylpyrrolidon in dem Koagulationsmittel 0 g/kg Koagulationsmittel betrug und der Anteil des Tocopherols in der Spinnmasse 0,00 % betrug. An den so sterilisierten Hohlfasermembranfiltern werden die Messmethoden zur Bestimmung des Zeta-Potentials, der Komplementaktivierung, des Platelet Loss und der Elution von Polyvinylpyrrolidon durchgeführt. Darüber hinaus werden sterilisierte Hohlfasermembranfilter einer beschleunigten Alterung bei 80°C für 30 bzw. 60 Tage unterzogen, wie in der "Messmethode zur Bestimmung der Elution von Polyvinylpyrrolidon" beschrieben. Nach der jeweiligen Alterung wurde die PVP-Elution bestimmt.

Zur Messung des Kontaktwinkels, des Gehalts von PVP in der Hohlfasermembran, des Gehalts von Polyvinylpyrrolidon in der oberflächennahen Schicht, und des Peakhöhenverhältnisses von $CNO^-$ und $SO_2^-$ mittels TOF-SIMS in der Oberflächenschicht werden entsprechend hergestellte Hohlfasermembranfilter geöffnet und Hohlfasermembranen zur Ermittlung der jeweiligen Werte entnommen. Die Ergebnisse der einzelnen Untersuchungen an Hohlfasermembranen, die nach dem Vergleichsbeispiel 2 gefertigt wurden, sind in Tabelle 1 dargestellt. Zusätzlich wurden die Albumin-Siebkoeffizienten nach 5 min, 10 min und 30 min bestimmt. Die Daten sind in Tabelle 2 angegeben. Weiter wurde nach obiger Vorschrift eine Membran hergestellt, jedoch mit den Dimensionen Lumendurchmesser 210 $\mu$m und Wandstärke 40 $\mu$m, welche zur Bestimmung des Plättchenverlustes nach der Absolutmethode verwendet wurde.

**Vergleichsbeispiel 3: Herstellung einer Vergleichs-Hohlfasermembran**

Es wurden für die Herstellung einer Vergleichs-Hohlfasermembran und der entsprechenden Filter die gleichen Ausgangsbedingungen wie in Beispiel 1 gewählt, mit dem Unterschied, dass der Anteil von Polyvinylpyrrolidon in dem Koagulationsmittel 5 g/kg Koagulationsmittel (5000 ppm) Gewichtsteile betrug und der Anteil des $\alpha$-Tocopherols in der Spinnmasse 0,01 % betrug. Während des Spinnprozesses kollabierte die Faser, so dass keine für die Messmethoden geeigneten Filter aufgebaut werden konnten. Auch Einzelfasermessungen (Kontaktwinkel) waren nicht möglich.

**Tabelle 1: Untersuchungsergebnisse der Beispiele 1 bis 7 und Vergleichsbeispiel 1 und 2**

| | PVP Elution nach 30 Tagen beschleunigter Alterung [$10^{*-7}$mg pro Einzelfaser] | PVP Elution nach 60 Tagen beschleunigter Allterung [$10^{*-7}$mg pro Einzelfaser] | PLT loss [%] | C5a Anstieg pro Stunde [%] | Zeta Potential [mV] | Kontaktwinkel [°] | PVP Gehalt in Faser [%] |
|---|---|---|---|---|---|---|---|
| **Bsp 1:** PVP 1000 ppm Vit E 0,01 % | | | 34 % | 30 % | -4 | | |
| **Bsp 2:** PVP 1500 ppm Vit E 0,01 % | 1100 | 1600 | 29 % | 18 % | -3,9 | 52 | 3,5 |
| **Bsp 3:** PVP 2500 ppm Vit E 0,01 % | | | 57 % | 17 % | -2,1 | | |
| **Bsp 4:** PVP 3000 ppm Vit E 0,05 % | 1000 | 1100 | 47 % | 8 % | -1,9 | | |
| **Bsp. 5:** PVP 1500 ppm. Vit. E 0,01 % PVP K90 im Fällm. | | | | | -2,97 | 45,4 | |

(fortgesetzt)

| | PVP Elution nach 30 Tagen beschleunigter Alterung [10*-7mg pro Einzelfaser] | PVP Elution nach 60 Tagen beschleunigter Allterung [10*-7mg pro Einzelfaser] | PLT loss [%] | C5a Anstieg pro Stunde [%] | Zeta Potential [mV] | Kontaktwinkel [°] | PVP Gehalt in Faser [%] |
|---|---|---|---|---|---|---|---|
| **Bsp. 6:** PVP 2000 ppm. Vit. E 0,01 % PVP K90 im Fällm. | | | | | -2,89 | 47,0 | |
| **Bsp. 7:** PVP 1000 ppm. Vit. E 0,01 % PVP K90 im Fällm. | | | | | -3,57 | 45,4 | |
| **Comp 1:** PVP 1500 ppm Vit E 0,00 % | 11000 | - (zu hohe Werte) | 29 % | 19 % | | | |
| **Comp 2:** PVP 0 ppm Vit E 0,00 % | 4200 | 5200 | 100 % | 100 % | -8,8 | 67 | 3,5 |

Die PVP-Elution wurde nach 30 bzw. 60 Tagen Alterung bei 80°C und < 5 % relativer Luftfeuchte bestimmt. Die Einheit dieser Messwerte ist $10^{-7}$ mg pro Einzelfaser. Die anderen Messwerte wurden an den ungealterten Proben ermittelt. Zusätzlich wurde der Kontaktwinkel an dem kommerziell erhältlichen Dialysator "Fresenius FX60" ermittelt. Der Wert wurde mit 64° bestimmt. In Tabelle 1 sind die Daten des Plättchenverlustes nach der Vergleichsmethode bestimmt.

**Gehalt an PVP in der oberflächennahen Schicht:**

| | |
|---|---|
| Beispiel 2: | 24,1 % |
| Beispiel 5: | 28,7 % |
| Beispiel 6: | 29,8 % |

(fortgesetzt)

| | |
|---|---|
| Beispiel 7: | 27,7 % |
| Vergleichsbeispiel 2: | 21,3 % |

**Peakhöhenverhältnis von $CNO^-$ und $SO_2^-$ mittels TOF-SIMS in einer Oberflächenschicht:**

| | |
|---|---|
| Beispiel 2: | 5,15 |
| Beispiel 5: | 6,50 |
| Beispiel 6: | 6,20 |
| Beispiel 7: | 5,20 |
| Vergleichsbeispiel 2: | 4,04 |

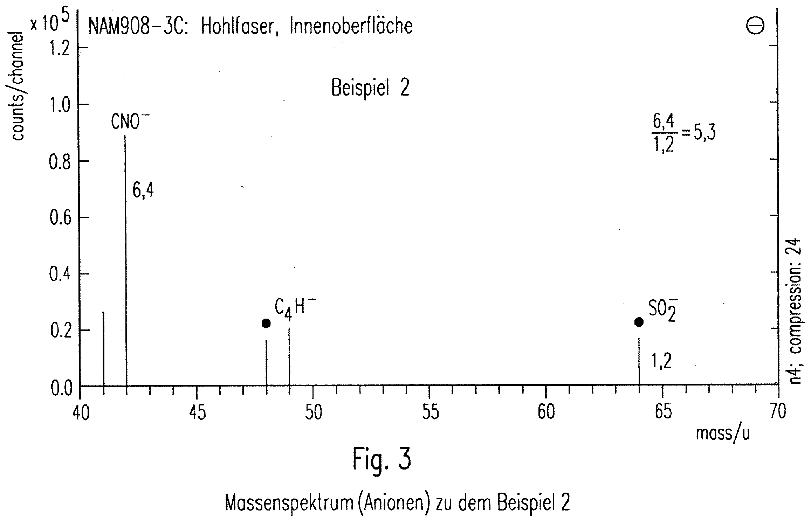

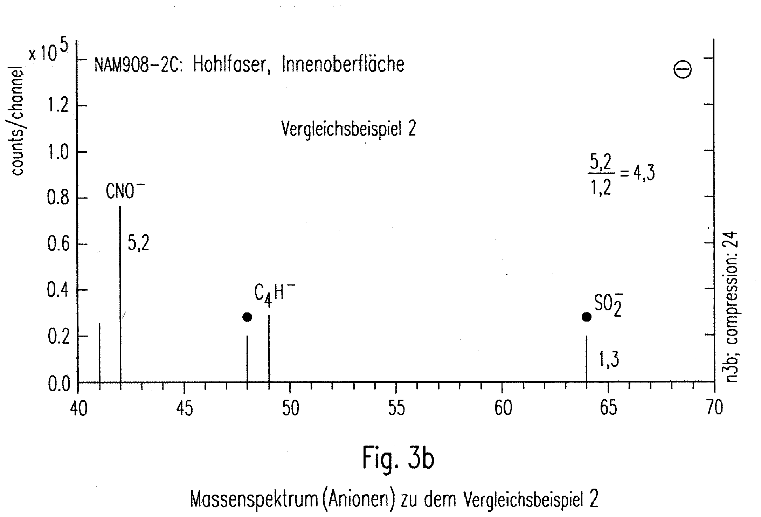

Das TOF-SIMS Spektrum (Anionen) des Beispiels 2 ist in Fig. 3a dargestellt, das TOF-SIMS Spektrum des Vergleichsbeispiels 2 ist in Fig. 3b dargestellt.

**Tabelle 2 Albuminsiebkoeffizienten der Beispiele 2 und 5 und des Vergleichsbeispiels 2**

| | Siebk (Sk). 5 min,. | Siebk. (Sk) 10 min | Siebk. (Sk) 30 min |
|---|---|---|---|
| Beispiel 2 | 0,0020 | 0,0016 | 0,0005 |
| Beispiel 5 | 0,0023 | 0,0017 | 0,0005 |
| Beispiel 6 | 0,0069 | 0,0020 | 0,0007 |

Es zeigt sich jeweils ein deutlicher Abfall des Siebkoeffizienten über die Zeit. Nach über 30 min stellt sich ein fast konstantes Gleichgewicht ein. Bevorzugt wird ein möglichst geringer Abfall des Albuminsiebkoeffizienten angestrebt, da solche Dialysatoren einen geringen anfänglichen Albuminverlust aufweisen. Deshalb wird auch das Verhältnis der Siebkoeffizenten nach 5 min,. und 10 min bzw. nach 5 min, und 30 min angegeben, was in Tabelle 3 dargestellt ist.

**Tabelle 3 Verhältnis der Siebkoeffizienten für die Beispiele 2 und 5 und das Vergleichsbeispiel 3**

| | Verhältnis Sk (5 min,) / Sk (10 min) | Verhältnis Sk (5 min,.) / Sk (30 min) |
|---|---|---|
| Beispiel 2 | 1,25 | 4,0 |
| Beispiel 5 | 1,35 | 4,6 |
| Vergleichsbeispiel 2 | 3,5 | 9,9 |

Das Vergleichsbeispiel weist einen deutlich höheren Abfall des Siebkoeffizienten zu Beginn des Tests auf. Das bedeutet, dass bei Dialysatoren, die einen gleichen Gleichgewichtssiebkoeffizienten nach längerer Testdauer aufweisen, erfindungsgemäße Membranen bzw. Dialysatoren einen deutlich geringeren anfänglichen Albuminverlust zeigen. Der Ernährungszustand eines mit einer erfindungsgemäßen Membran bzw. Dialysator behandelten Patienten wird somit verbessert.

Daten aus der Bestimmung des Plättchenverlustes bestimmt nach der Absolutmethode:

**Tabelle 4:**

| | Plättchenverlust ( %) | Standardabw. ( %) |
|---|---|---|
| Beispiel 2 | 18,6 | 3,1 |
| Vergleichsbeispiel 2 | 66,8 | 14,3 |

Für die Ermittlung der Daten wurden für das Beispiel 2 68 Tests und für Vergleichsbeispiel 2 22 Tests durchgeführt und aus diesen Messungen jeweils der Mittelwert gebildet. Es zeigt sich, dass erfindungsgemäße Membrane einen deutlich geringeren Plättchenverlust, gemessen nach der Methode "Bestimmung des "Platelet Loss" (Absolutmethode)", aufweisen und somit hemokompatibler sind.

## Patentansprüche

1. Verfahren zur Herstellung einer Hohlfasermembran, aufweisend die folgenden Schritte:

   • Bereitstellen mindestens einer Spinnmasse aufweisend Polysulfon und Polyvinylpyrrolidon, zumindest ein aprotisches polares Lösungsmittel und ein α-Tocopherol, insbesondere ein Tocotrienol,
   • Bereitstellen mindestens eines Koagulationsmittels aufweisend zumindest ein aprotisches polares Lösungsmittel, und/oder mindestens ein Nicht-Lösemittel, insbesondere Wasser,
   • Fördern der Spinnmasse durch wenigstens einen Ringspalt einer Spinndüse zu einem hohlen Spinnfaden,
   • Fördern des Koagulationsmittels durch eine zentrale Bohrung der Spinndüse in das Lumen des Spinnfadens,
   • Einleiten des Spinnfadens in ein Fällbad,

   **dadurch gekennzeichnet, dass** die Spinnmasse 0,001 bis 0,05 Gew.% des α-Tocopherols, oder insbesondere des Tocotrienols, enthält und dass weiterhin das Koagulationsmittel 0,5 bis 4 g Polyvinylpyrrolidon pro kg des Koagulationsmittelsenthält.

2. Verfahren nach Anspruch 1, **dadurch gekennzeichnet, dass** die Spinnmasse 2 bis 7 Gew.%, insbesondere 3 bis 5 Gew.%, Polyvinylpyrrolidon, bezogen auf die Gesamtmasse der Spinnmasse, enthält.

3. Verfahren nach einem der voranstehenden Ansprüche, **dadurch gekennzeichnet, dass** das im Koagulationsmittel enthaltene Polyvinylpyrrolidon, eine Molekulargewichtsverteilung im Bereich von 200.000 g/mol bis 2.000.000 g/mol, insbesondere ein gewichtsmittleres Molekulargewicht von 900.000 g/mol aufweist.

4. Verfahren nach einem der voranstehenden Ansprüche, **dadurch gekennzeichnet, dass** das gewichtsmittlere Molekulargewicht (Mw) des PVP im Koagulationsmittel höher ist als das des PVP in der Spinnmasse.

5. Verfahren nach Anspruch 4, wobei das gewichtsmittlere Molekulargewicht (Mw) des PVP in der Spinnmasse unter 1.000.000 g/mol beträgt und das gewichtsmittlere Molekolargewicht (Mw) des PVP in dem Koagulationsmittel über 1.000.000 g/mol beträgt.

6. Hohlfasermembran, aufweisend ein Membranmaterial, das Polysulfon, Polyvinylpyrrolidon und α-Tocopherol, insbesondere Tocotrienol aufweist, **dadurch gekennzeichnet, dass** das α-Tocopherol oder Tocotrienol in einem Anteil von 0,005 bis 0,25 Gew. % bezogen auf das Gesamtgewicht der Hohlfasermembran vorhanden ist.

7. Hohlfasermembran nach Anspruch 6, **dadurch gekennzeichnet, dass** die Elution des Polyvinylpyrrolidons gemessen nach der "Messmethode zur Bestimmung der Elution von Polyvinylpyrrolidon" gemäß der Beschreibung, nach einem Lagerzeitraum von 30 Tagen bei 80°C und einer relativen Luftfeuchte von < 5 %, weniger als $4000*10^{-7}$ mg/ pro Einzelfaser, insbesondere nach 60 Tagen bei 80°C und einer relativen Luftfeuchte von < 5 % weniger als $5000*10^{-7}$ mg/ Einzelfaser beträgt.

8. Hohlfasermembran nach Anspruch 6, **dadurch gekennzeichnet, dass** die Hohlfasermembran auf der lumenseitigen Oberfläche ein Zeta-Potential gemessen nach der "Messmethode zur Bestimmung des Zeta Potentials" gemäß der Beschreibung von -1 mV bis weniger als -7mV, insbesondere -1 mV bis -5 mV, weiter insbesondere -1 bis -4 mV, aufweist.

9. Hohlfasermembran nach einem der Ansprüche 6 bis 8, **dadurch gekennzeichnet, dass** der Gehalt von Polyvinylpyrrolidon in der oberflächennahen Schicht des inneren Lumens der Membran gemäß Messmethode XPS 22 Gew.% oder mehr beträgt.

10. Hohlfasermembran nach einem der Ansprüche 6 bis 9, **dadurch gekennzeichnet, dass** das Peakhöhenverhältnis von $CNO^-$ und $SO_2^-$, bestimmt mittels TOF-SIMS an der Oberflächenschicht des inneren Lumens der Membran, 4,5 oder mehr beträgt.

11. Hohlfasermembran nach einem der Ansprüche 6-10,
    **dadurch gekennzeichnet, dass** die

Hohlfasermembran einen Gehalt an Polyvinylpyrrolidon von 3 bis 5 % (w/w) aufweist.

**12.** Hohlfasermembran nach einem der Ansprüche 6 bis 11, wobei das Verhältnis der Siebkoeffizienten für Albumin, gemessen nach 5 min, zu dem Siebkoeffizienten gemessen nach 30 min, gemäß der Messmethode der Beschreibung, weniger als 7, insbesondere weniger als 5 beträgt.

**13.** Hohlfasermembran nach einem der Ansprüche 6 bis 12, wobei das Verhältnis der Siebkoeffizienten für Albumin, gemessen nach 5 min, zu dem Siebkoeffizienten gemessen nach 10 min, gemäß der Messmethode der Beschreibung, weniger als 3, insbesondere weniger als 2 beträgt.

**14.** Hohlfasermembran nach einem der Ansprüche 6 bis 13, **dadurch gekennzeichnet, dass** der Plättchenverlust, gemessen nach der Methode "Bestimmung des "Platelet Loss" (Absolutmethode)", weniger als 50 %, bevorzugt weniger als 30 %, besonders bevorzugt weniger als 20 % beträgt.

**15.** Hohlfasermembranfilter, insbesondere Dialysator für die Hämodialyse, aufweisend eine Vielzahl von Hohlfasermembranen, nach einem der Ansprüche 6 bis 14 oder hergestellt nach dem Verfahren gemäß einem der Ansprüche 1 bis 5.

## Claims

**1.** Method for manufacturing a hollow fiber membrane comprising the following steps:

- providing at least one spin mass comprising polysulfone and polyvinylpyrrolidone, at least one aprotic polar solvent and an $\alpha$-Tocopherol, in particular a tocotrienol,
- providing at least one coagulant comprising at least one aprotic polar solvent and/or at least one non-solvent, in particular water,
- conveying the spin mass through at least one annular gap of a spinneret into a hollow spinning yarn,
- conveying the coagulant through a central bore of the spinneret into the lumen of the spinning yarn,
- introducing the spinning yarn into a precipitating bath,

**characterized in that** the spin mass contains 0.001 to 0.05% by weight of the $\alpha$-Tocopherol, in particular the tocotrienol, and the coagulant furthermore comprises 0.5 g to 4 g polyvinylpyrrolidone per kg coagulant.

**2.** The method according to claim 1, **characterized in that** the spin mass contains 2 to 7% by weight, in particular 3 to 5% by weight, polyvinyl-pyrrolidone relative to the total mass of the spin mass.

**3.** The method according to any of the preceding claims, **characterized in that** the polylvinylpyrrolidone, contained in the coagulant has a molecular weight distribution in the range of 200,000 g/mol to 2,000,000 g/mol, in particular a weight-average molecular weight of 900,000 g/mol.

**4.** The method according to any of the preceding claims, **characterized in that** the weight average molecular weight (Mw) of the polyvinylpyrrolidone (PVP) in the coagulating agent is higher than that of the PVP in the spinning mass

**5.** The method according to claim 4, wherein the weight average molecular weight (Mw) of the PVP in the spinning mass is below 1,000,000 g/mol and the weight average molecular weight (Mw) of the PVP in the coagulating agent is above 1,000,000 g/mol.

**6.** Hollow fiber membrane having a membrane material comprising polysulfone, polyvonylpyrrolidone as well as $\alpha$-Tocopherol, in particular tocotrienol, **characterized in that** the $\alpha$-Tocopherol, in particular tocotrienol is present at a 0.005 to 0.25% by weight ratio in relation to the total weight of the hollow fiber membrane.

**7.** The hollow fiber membrane according to claim 6, **characterized in that** the elution of the polyvinylpyrrolidone, measured according to the "Messmethode zur Bestimmung der Elution von Polyvinylpyrrolidon" of the description amounts to less than $4000*10^{-7}$ mg per individual fiber after being stored for a period of 30 days at 80°C and <5% relative humidity, in particular less than $5000*10^{-7}$ mg per individual fiber after 60 days at 80°C and <5% relative humidity.

8. The hollow fiber membrane according to claim 6, **characterized in that** the hollow fiber membrane has a zeta potential on the lumen-side surface of -1 mV to less than -7 mV, in particular -1 mV to -5 mV, further particularly -1 mV to -4 mV.

9. The hollow fiber membrane according to any of claims 6 to 8, **characterized in that** the polyvinylpyrrolidone content in the near-surface layer of the inner lumen of the membrane amounts to 22% by weight or more pursuant to an XPS measurement method.

10. The hollow fiber membrane according to one of claims 6 to 9, **characterized in that** the peak height ratio of $CNO^-$ and $SO_2^-$ amounts to 4.5 or higher on the surface layer of the inner lumen of the membrane as determined by TOF-SIMS.

11. The hollow fiber membrane according to any of claims 6 to 10, **characterized in that** the hollow fiber membrane has a polyvinylpyrrolidone content of 3 to 5% (w/w).

12. The hollow fiber membrane according to any of claims 6 to 11, wherein the ratio of the sieving coefficients for albumin measured after 5 min to the sieving coefficient measured after 30 min according to the measurement method of the description is less than 7, in particular less than 5.

13. The hollow fiber membrane according to any of claims 6 to 12, wherein the ratio of the sieving coefficients for albumin, measured after 5 min, to the sieving coefficient, measured after 10 min, according to the measurement method of the description, is less than 3, in particular less than 2.

14. The hollow fiber membrane according to any one of claims 6 to 13, **characterized in that** the platelet loss measured according to the method " Bestimmung des "Platelet Loss" (Absolutmethode)" is less than 50%, preferably less than 30%, particularly preferably less than 20%.

15. Hollow fiber membrane filter, in particular dialyzer for hemodialysis, comprising a plurality of hollow fiber membranes according to any of claims 6 to 14 or manufactured according to any of claims 1 to 5.

## Revendications

1. Procédé de fabrication d'une membrane à fibres creuses, présentant les étapes suivantes de :

   - fourniture d'au moins une masse de filage présentant de la polysulfone et de la polyvinylpyrrolidone, au moins un solvant aprotique polaire et un $\alpha$-tocophérol, en particulier un tocotriénol,
   - fourniture d'au moins un agent de coagulation présentant au moins un solvant aprotique polaire et/ou au moins un non solvant, en particulier de l'eau,
   - passage de la masse de filage à travers au moins une fente circulaire d'une filière pour donner un fil filé creux,
   - passage de l'agent de coagulation à travers un trou central de la filière dans la lumière du fil filé,
   - introduction du fil filé dans un bain de précipitation,

   **caractérisé en ce que** la masse de filage contient 0,001 à 0,05 % en poids de l'a-tocophérol, ou en particulier du tocotriénol, et **en ce que**, en outre, l'agent de coagulation contient 0,5 à 4 g de polyvinylpyrrolidone par kg de l'agent de coagulation.

2. Procédé selon la revendication 1, **caractérisé en ce que** la masse de filage contient de 2 à 7 % en poids, en particulier de 3 à 5 % en poids, de polyvinylpyrrolidone, par rapport à la masse totale de la masse de filage.

3. Procédé selon l'une des revendications précédentes, **caractérisé en ce que** la polyvinylpyrrolidone contenue dans l'agent de coagulation présente une distribution de poids moléculaire dans la plage de 200 000 g/mol à 2 000 000 g/mol, en particulier un poids moléculaire moyen en poids de 900 000 g/mol.

4. Procédé selon l'une des revendications précédentes, **caractérisé en ce que** le poids moléculaire moyen en poids (Pm) de la PVP dans l'agent de coagulation est supérieur à celui de la PVP dans la masse de filage.

5. Procédé selon la revendication 4, dans lequel le poids moléculaire moyen en poids (Pm) de la PVP dans la masse de filage est inférieur à 1 000 000 g/mol et le poids moléculaire moyen en poids (Pm) de la PVP dans l'agent de coagulation est supérieur à 1 000 000 g/mol.

**6.** Membrane à fibres creuses, présentant une matière de membrane qui comprend de la polysulfone, de la polyvinylpyrrolidone et de l'a-tocophérol, en particulier du tocotriénol, **caractérisé en ce que** l'a-tocophérol ou le tocotriénol est présent en une proportion de 0,005 à 0,25 % en poids par rapport au poids total de la membrane à fibres creuses.

**7.** Membrane à fibres creuses selon la revendication 6, **caractérisée en ce que** l'élution de la polyvinylpyrrolidone, mesurée d'après la « méthode de mesure pour la détermination de l'élution de la polyvinylpyrrolidone » selon la description, après une période de stockage de 30 jours à 80 °C et à une humidité relative de l'air de < 5 %, est inférieure à $4000*10^{-7}$ mg/par fibre individuelle, en particulier après 60 jours à 80 °C et à une humidité relative de l'air de < 5 %, est inférieure à $5000*10^{-7}$ mg/par fibre individuelle.

**8.** Membrane à fibres creuses selon la revendication 6, **caractérisée en ce que** la membrane à fibres creuses présente sur la surface du côté lumière un potentiel zêta, mesuré d'après la « méthode de mesure pour la détermination du potentiel zêta » selon la description, de -1 mV à moins de -7 mV, en particulier de -1 mV à -5 mV, en outre, en particulier de -1 à -4 mV.

**9.** Membrane à fibres creuses selon l'une des revendications 6 à 8, **caractérisée en ce que** la teneur en polyvinylpyrrolidone dans la couche proche de la surface de la lumière interne de la membrane, selon la méthode de mesure XPS, est de 22 % en poids ou plus.

**10.** Membrane à fibres creuses selon l'une des revendications 6 à 9, **caractérisée en ce que** le rapport de hauteurs de pic de $CNO^-$ et $SO_2^-$, déterminé par TOF-SIMS sur la couche de surface de la lumière interne de la membrane, est de 4,5 ou plus.

**11.** Membrane à fibres creuses selon l'une des revendications 6 à 10, **caractérisée en ce que** la membrane à fibres creuses présente une teneur en polyvinylpyrrolidone de 3 à 5 % (p/p).

**12.** Membrane à fibres creuses selon l'une des revendications 6 à 11, dans laquelle le rapport du coefficient de filtrage pour l'albumine, mesuré au bout de 5 min, sur le coefficient de filtrage mesuré au bout de 30 min, d'après la méthode de mesure de la description, est inférieur à 7, en particulier inférieur à 5.

**13.** Membrane à fibres creuses selon l'une des revendications 6 à 12, dans laquelle le rapport du coefficient de filtrage pour l'albumine, mesuré au bout de 5 min, sur le coefficient de filtrage mesuré au bout de 10 min, d'après la méthode de mesure de la description, est inférieur à 3, en particulier inférieur à 2.

**14.** Membrane à fibres creuses selon l'une des revendications 6 à 13, **caractérisée en ce que** la perte de plaquettes, mesurée d'après la méthode « détermination de la « perte de plaquettes » (méthode absolue) », est inférieure à 50 %, de préférence inférieure à 30 %, particulièrement préférentiellement inférieure à 20 %.

**15.** Membrane à fibres creuses, en particulier dialyseur pour l'hémodialyse, présentant une multitude de membranes à fibres creuses, selon l'une des revendications 6 à 14, ou fabriquées d'après le procédé selon l'une des revendications 1 à 5.

8
11
6
P
7
1
2
12
5
10
P
9
3
4

Fig. 1
Anordnung zum Test eines Dialysators

8
Overhead
tank
6
Keithley 2000
Multimeter
7
Differential
Pressure Gauge
4a
5
5a
2
4
3
1
3a
2a
4-way valve

Fig. 2

Versuchsaufbau zur Messung des Zeta-Potentials von Hohlfasermembranen

3/3a
Pressure
gauge port
4/4a
5/5a
Electrode
2/2a

Fig. 2a

Versuchsaufbau zur Messung des Zeta-Potentials von Hohlfasermembranen

NAM908–3C: Hohlfaser, Innenoberfläche
Beispiel 2
counts/channel
x 10^5
1.2
1.0
0.8
0.6
0.4
0.2
0.0
40
45
50
55
60
65
70
mass/u
CNO⁻
6,4
C₄H⁻
SO₂⁻
1,2
6,4/1,2 = 5,3
n4; compression: 24

Fig. 3

Massenspektrum (Anionen) zu dem Beispiel 2

NAM908-2C: Hohlfaser, Innenoberfläche
Vergleichsbeispiel 2
n3b; compression: 24
counts/channel
x 10^5
1.2
1.0
0.8
0.6
0.4
0.2
0.0
40
45
50
55
60
65
70
CNO⁻
5,2
C4H⁻
SO2⁻
1,3
5,2 / 1,2 = 4,3

Fig. 3b

Massenspektrum (Anionen) zu dem Vergleichsbeispiel 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0168783 A
- EP 0850678 B1
- EP 0568045 A
- EP 2987514 A1
- EP 2737916 A1
- EP 0749775 A1
- EP 2719408 A1
- DE 102016224627


### In der Beschreibung aufgeführte Nicht-Patentliteratur


- **MARCEL MULDER.** Principles of Membrane Technology. Kluwer Academic Publisher, 1996
- **A. ERLENKÖTTER ; P. ENDRES ; B. NEDERLOF ; C. HORNIG ; J. VIENKEN.** *Artificial Organs,* 2008, vol. 32 (12), 962
- **ERLENKÖTTER et al.** Score Model for the Evaluation of Dialysis Membrane Hemocompatibility. *Artificial Organs,* 2008, vol. 32 (12), 962-998